# EUROPEAN PATENT SPECIFICATION

(11) **EP 2 001 556 B1**
(45) Date of publication and mention of the grant of the patent: **01.10.2014**
(21) Application number: 07712962.5
(22) Date of filing: 28.03.2007
(51) Int. Cl.: A61P 25/28, A61K 31/5415

(54) **THIONINIUM COMPOUNDS AND THEIR USE**
THIONINIUM-VERBINDUNGEN UND IHRE VERWENDUNG
COMPOSÉS DE THIONINIUM ET UTILISATION

(30) Priority: 29.03.2006 US 786699 P
(43) Date of publication of application: 17.12.2008
(73) Proprietor: Wista Laboratories Ltd., Singapore 139948 (SG)
(72) Inventor: WISCHIK, Claude, Michel, Scotland (GB); RICKARD, Janet, Elizabeth, Scotland (GB); HARRINGTON, Charles, Robert, Scotland (GB); HORSLEY, David, Scotland (GB); STOREY, John, Mervyn, David, Scotland (GB); MARSHALL, Colin, Scotland (GB); SINCLAIR, James, Peter, Scotland (GB)
(74) Representative: Wytenburg, Wilhelmus Johannes
(86) International application number: PCT/GB2007/001107
(87) International publication number: WO 2007/110630

(56) References cited:
- WO-A-96/04915
- WO-A-96/30766
- WO-A-02/075318
- WO-A-2005/030676
- WO-A-2006/032879
- US-A1- 2006 287 523
- BUÉE L ET AL: "Comparative biochemistry of tau in progressive supranuclear palsy, corticobasal degeneration, FTDP-17 and Pick's disease.", BRAIN PATHOLOGY (ZURICH, SWITZERLAND) OCT 1999 LNKD- PUBMED:10517507, vol. 9, no. 4, October 1999 (1999-10), pages 681-693, ISSN: 1015-6305

## Description

This application is related to United States patent application number 60/786,699 filed 29 March 2006.

### TECHNICAL FIELD

This invention pertains generally to processes, uses, methods and materials utilising particular diaminophenothiazinium compounds. These compounds are useful as drugs, for example, in the treatment of tauopathies, such as Alzheimer's disease.

### BACKGROUND

A number of patents and publications are cited herein in order to more fully describe and disclose the invention and the state of the art to which the invention pertains.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Thus, for example, reference to "a pharmaceutical carrier" includes mixtures of two or more such carriers, and the like.

Ranges are often expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

Conditions of dementia such as Alzheimer's disease (AD) are frequently characterised by a progressive accumulation of intracellular and/or extracellular deposits of proteinaceous structures such as β-amyloid plaques and neurofibrillary tangles (NFTs) in the brains of affected patients. The appearance of these lesions largely correlates with pathological neurofibrillary degeneration and brain atrophy, as well as with cognitive impairment (see, e.g., Mukaetova-Ladinska, E.B., et al., 2000, Am. J. Pathol., Vol. 157, No. 2, pp. 623-636).

In AD, both neuritic plaques and NFTs contain paired helical filaments (PHFs), of which a major constituent is the microtubule-associated protein tau (see, e.g., Wischik et al., 1988, PNAS USA, Vol. 85, pp. 4506-4510). Plaques also contain extracellular β-amyloid fibrils derived from the abnormal processing of amyloid precursor protein (APP) (see, e.g., Kang et al., 1987, Nature, Vol. 325, p. 733). An article by Wischik et al. (in 'Neurobiology of Alzheimer's Disease', 2nd Edition, 2000, Eds. Dawbarn, D. and Allen, S.J., The Molecular and Cellular Neurobiology Series, Bios Scientific Publishers, Oxford) discusses in detail the putative role of tau protein in the pathogenesis of neurodegenerative dementias. Loss of the normal form of tau, accumulation of pathological PHFs, and loss of synapses in the mid-frontal cortex all correlate with associated cognitive impairment. Furthermore, loss of synapses and loss of pyramidal cells both correlate with morphometric measures of tau-reactive neurofibrillary pathology, which parallels, at a molecular level, an almost total redistribution of the tau protein pool from a soluble to a polymerised form (i.e., PHFs) in Alzheimer's disease.

Tau exists in alternatively-spliced isoforms, which contain three or four copies of a repeat sequence corresponding to the microtubule-binding domain (see, e.g., Goedert, M., et al., 1989, EMBO J., Vol. 8, pp. 393-399; Goedert, M., et al., 1989, Neuron, Vol. 3, pp. 519-526). Tau in PHFs is proteolytically processed to a core domain (see, e.g., Wischik, C.M., et al., 1988, PNAS USA, Vol. 85, pp. 4884-4888; Wischik et al., 1988, PNAS USA, Vol. 85, pp. 4506-4510; Novak, M., et al., 1993, EMBO J., Vol. 12, pp. 365-370) which is composed of a phase-shifted version of the repeat domain; only three repeats are involved in the stable tau-tau interaction (see, e.g., Jakes, R., et al., 1991, EMBO J., Vol. 10, pp. 2725-2729). Once formed, PHF-like tau aggregates act as seeds for the further capture and provide a template for proteolytic processing of full-length tau protein (see, e.g., Wischik et al., 1996, PNAS USA, Vol. 93, pp. 11213-11218).

The phase shift which is observed in the repeat domain of tau incorporated into PHFs suggests that the repeat domain undergoes an induced conformational change during incorporation into the filament. During the onset of AD, it is envisaged that this conformational change could be initiated by the binding of tau to a pathological substrate, such as damaged or mutated membrane proteins (see, e.g., Wischik, C.M., et al., 1997, in "Microtubule-associated proteins: modifications in disease", Eds. Avila, J., Brandt, R. and Kosik, K. S. (Harwood Academic Publishers, Amsterdam) pp. 185-241).

In the course of their formation and accumulation, PHFs first assemble to form amorphous aggregates within the cytoplasm, probably from early tau oligomers which become truncated prior to, or in the course of, PHF assembly (see, e.g., Mena, R., et al., 1995, Acta Neuropathol., Vol. 89, pp. 50-56; Mena, R., et al., 1996, Acta Neuropathol., Vol. 91, pp. 633-641). These filaments then go on to form classical intracellular NFTs. In this state, the PHFs consist of a core of truncated tau and a fuzzy outer coat containing full-length tau (see, e.g., Wischik et al., 1996, PNAS USA, Vol. 93, pp. 11213-11218). The assembly process is exponential, consuming the cellular pool of normal functional tau and inducing new tau synthesis to make up the deficit (see, e.g., Lai, R. Y. K., et al., 1995, Neurobiology of Ageing, Vol. 16, No. 3, pp. 433-445). Eventually, functional impairment of the neurone progresses to the point of cell death, leaving behind an extracellular NFT. Cell death is highly correlated with the number of extracellular NFTs (see, e.g., Wischik et al., in 'Neurobiology of Alzheimer's Disease', 2nd Edition, 2000, Eds. Dawbarn, D. and Allen, S.J., The Molecular and Cellular Neurobiology Series, Bios Scientific Publishers, Oxford). As tangles are extruded into the extracellular space, there is progressive loss of the fuzzy outer coat of the neurone with corresponding loss of N-terminal tau immunoreactivity, but preservation of tau immunoreactivity associated with the PHF core (see, e.g., Bondareff, W. et al., 1994, J. Neuropath. Exper. Neurol., Vol. 53, No. 2, pp. 158-164).

Diaminophenothiazines have previously been shown to inhibit tau protein aggregation and to disrupt the structure of PHFs, and reverse the proteolytic stability of the PHF core (see, e.g., WO 96/30766, F Hoffman-La Roche). Such compounds were disclosed for use in the treatment or prophylaxis of various diseases, including Alzheimer's disease. These included, amongst others:

| | |
|---|---|
| MTC | 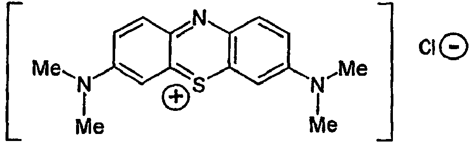 |
| | methyl-thioninium chloride |
| DMMTC | 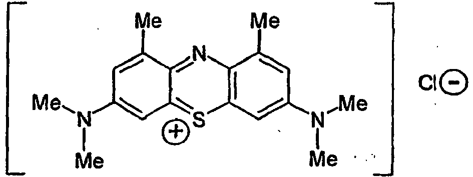 |
| | 1,9-dimethyl-methyl-thioninium chloride |

Additionally, WO 02/055720 (The University Court of the University of Aberdeen) discusses the use of reduced forms of diaminophenothiazines specifically for the treatment of a variety of protein aggregating diseases, although the disclosure is primarily concerned with tauopathies.

WO 2005/030676 (The University Court of the University of Aberdeen) discusses radiolabelled phenothiazines, and their use in diagnosis and therapy, for example, of tauopathies.

Notwithstanding these disclosures, it will be appreciated that the provision of one or more further compounds, not previously specifically identified as being effective tau protein aggregation inhibitors, would provide a contribution to the art.

PCT/GB2005/003634 (TauRx Therapeutics Pte. Ltd) was filed, but not published, prior to the filing of the present application (WO 2006/032879 A2 was published on 30 March 2006). That application relates *inter alia* to methods of synthesizing and purifying certain 3,7-diamino-phenothiazin-5-ium compounds (referred to as "diaminophenothiazinium compounds") including MTC and other compounds described herein. It further discloses therapeutic uses of these compounds.

WO 02/075318 A2 (26 September 2002) describes methods relating generally to the labelling and detection of neurofibrillary tangles, as well as ligands (such as Toluidine Blue O, Thionine, Azure A, Azure B, 1,9-Dimethyl-Methylene Blue (DMMTC), and Methylene Blue (MTC)) for diagnosis, prognosis, and treatment of diseases such as Alzheimer's Disease.

WO 2005/030676 A1 (07 April 2005) describes methods for preparing radiolabelled phenothiazines (such as Toluidine Blue O, Thionine, Azure A, Azure B, and Methylene Blue (MTC)), and their use in diagnosis and therapy, for example, of tauopathies.

WO 96/30766 A1 (03 October 1996) describes methods of screening for compounds capable of modulating or inhibiting pathological tau-tau protein association and pathological neurofilament aggregation (such as Toluidine Blue O, Thionine, Azure A, Azure B, 1,9-Dimethyl-Methylene Blue (DMMTC) which are useful in treatment of conditions such as Alzheimer's Disease.

WO 96/04915 A1 (22 February 1996) describes methods for treating and/or preventing Alzheimer's Disease using phenothiazines and/or thioxanthenes.

US 2006/0287523 A1 (21 December 2006) is a US application largely corresponding to the present application.

WO 2006/032879 A2 (30 March 2006) describes method for the chemical synthesis and purification of diaminophenothiazinium compounds (including Ethylthioninium Chloride (ETC), 1,9-Diethyl Methylthioninium Chloride (DEMTC), and Ethylthioninium Chloride (ETC) Zinc Chloride (Double Salt)) and their use to treat, for example, Alzheimer's disease.

### DESCRIPTION OF THE INVENTION

The present inventors have now identified certain thioninium compounds as being effective tau protein aggregation inhibitors and in preferred forms having certain other desirable properties, for example by comparison with the compounds of the prior art discussed above.

As discussed above, tau proteins are characterised as being one among a larger number of protein families which co-purify with microtubules during repeated cycles of assembly and disassembly (Shelanski et al. (1973) Proc. Natl. Acad. Sci. USA, 70., 765-768), and are known as microtubule-associated-proteins (MAPs). Members of the tau family share the common features of having a characteristic N-terminal segment, sequences of approximately 50 amino acids inserted In the N-terminal segment, which are developmentally regulated In the brain, a characteristic tandem repeat region consisting of 3 or 4 tandem repeats of 31-32 amino acids, and a C-terminal tail.

One or more of these specific compounds are known in the art - for example MTZ is described in Fierz-David and Blangley, 1949, "F. Oxazine and Thiazine Dyes," in: Fundamental Processes of Dye Chemistry, published by Interscience (London, UK), pp. 308-314. However it is believed that none of these have previously been disclosed In the prior art as tau protein aggregation inhibitors.

Described herein are methods, uses, compositions and other materials employing these compounds as tau protein aggregation inhibitors and as therapeutics or prophylactics of diseases associated with tau protein aggregation ("tauopathies"). Also described herein are processes for making these compounds.

### Compounds

In general, the present invention relates to one or more compounds selected from the following diaminophenothiazinium compounds, wherein (*) denotes a reference compound:

| | | |
|---|---|---|
| A | ETC | 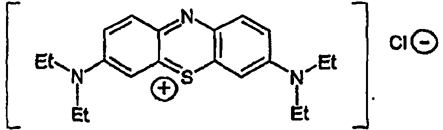 |
| | | ethyl-thioninium chloride |
| B | DEMTC | 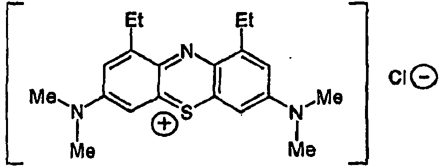 |
| | | 1,9-diethyl-methyl-thioninium chloride |
| C | DMETC | 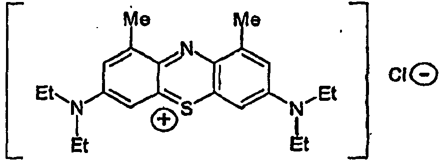 |
| | | 1,9-dimethyl-ethyl-thioninium chloride |
| D | DEETC | 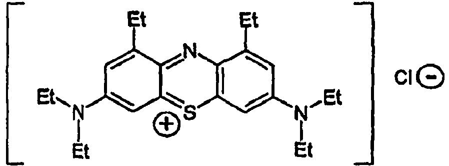 |
| | | 1,9-diethyl-ethyl-thioninium chloride |
| E (*) | MTZ | 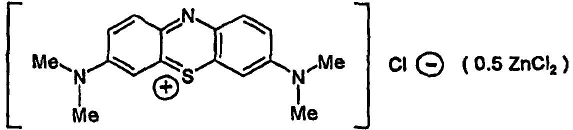 |
| | | methyl-thioninium chloride zinc chloride mixed salt |
| F | ETZ | 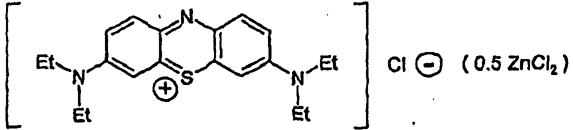 |
| | | ethyl-thioninium chloride zinc chloride mixed salt |
| G (*) | MTI | 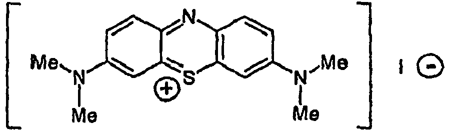 |
| | | methyl-thioninium iodide |
| H (*) | MTI.HI | 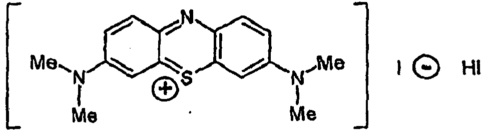 |
| | | methyl-thioninium iodide hydrogen iodide mixed salt |
| I | ETI | 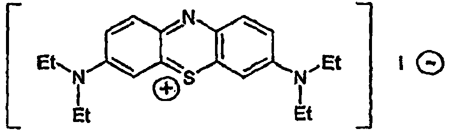 |
| | | ethyt-thioninium iodide |
| J | ETI.HI | 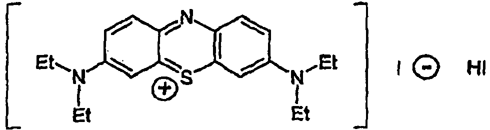 |
| | | ethyl-thioninium iodide hydrogen iodide mixed salt |
| K (*) | MTN | 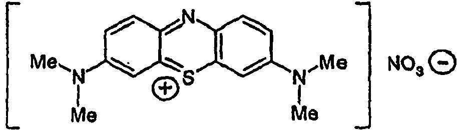 |
| | | methyl-thioninium nitrate |
| L | ETN | 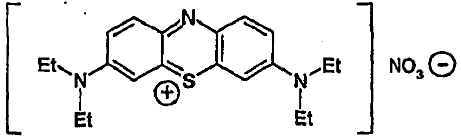 |
| | | ethyl-thioninium nitrate |

These compounds are described herein as "diaminophenothiazinium compounds" or "DAPT compounds" or "compounds of the invention" or (unless context demands otherwise) "active compounds".

### Isotopic Variation

In one embodiment, one or more of the carbon atoms of the compound is ¹¹C or ¹³C or ¹⁴C.

In one embodiment, one or more of the carbon atoms of the compound is ¹¹C.

In one embodiment, one or more of the carbon atoms of the compound is ¹³C.

In one embodiment, one or more of the carbon atoms of the compound is ¹⁴C.

In one embodiment, one or more of the nitrogen atoms of the compound is ¹⁵N.

In one embodiment, one or more or all of the carbon atoms of one or more or all of the groups -Me (or -Et) is ¹¹C.

In one embodiment, one or more or all of the carbon atoms of one or more or all of the groups -NMe₂ (or -NEt₂) is ¹¹C.

In one embodiment, the groups -NMe₂ are -N(¹¹CH₃)₂.

*Uses to reverse or inhibit the aggregation of tau protein.*

Described herein is the use of a diaminophenothiazinium compound to reverse or inhibit the aggregation of tau protein. This aggregation may be *in vitro,* or *In vivo,* and may be associated with a tauopathy disease state as discussed herein. Also described are methods of reversing or inhibiting the aggregation of tau protein comprising contacting the aggregate or protein with a compound as described herein.

### Preferred compounds

In this and all other aspects of the invention, unless context demands otherwise, the compound is selected from the list consisting of A, B, C, D, F, I, K, and L (in each case optionally being an isotopic variant thereof as described above).

In one embodiment, it is compound A.

In one embodiment, it is compound B.

In one embodiment, it is compound C.

In one embodiment, It is compound D.

In one embodiment, it is compound F.

In one embodiment, it is compound I.

In one embodiment, it is compound J.

In one embodiment, it is compound L.

In one embodiment the diaminophenothiazinium compound may be one which is *obtained by, or is obtainable by,* a method as described herein (see "Methods of Synthesis" below).

Preferred compounds are those which show high activity in the assays described herein, particularly the *in vitro* assay described below. Preferred compounds have a B50 of less than 500, more preferably less than 300, 200, 100, 90, 80, 70, 60, 50,40, 30 or 20 µM, as determined with reference to the Examples herein.

In one embodiment the diaminophenothiazinium compound has a RxIndex (RxI) value obtained as determined with reference to the Examples herein of greater than or equal to 150, more preferably greater than or equal to 160, 170, 180, 190, 200, 500, 1000, 1500, or 2000.

### Methods of treatment or prophylaxis and 1^{st} & 2^{nd} medical uses

Described herein is a method of treatment or prophylaxis of a tauopathy condition in a patient, comprising administering to said patient a therapeutically-effective amount of a diaminophenothiazinium compound, as described herein.

Aspects of the present invention relate to "tauopathies". As well as Alzheimer's disease (AD), the pathogenesis of neurodegenerative disorders such as Pick's disease and Progressive Supranuclear Palsy (PSP) appears to correlate with an accumulation of pathological truncated tau aggregates in the dentate gyrus and stellate pyramidal cells of the neocortex, respectively. Other dementias include fronto-temporal dementia (FTD); fronto-temporal dementia with parkinsonism linked to chromosome 17 (FTDP-17); disinhibition-dementla-parkinsonism-amyotrophy complex (DDPAC); pallido-ponto-nigral degeneration (PPND); Guam-ALS syndrome; pallido-nigro-luyslan degeneration (PNLD); cortico-basal degeneration (CBD) and others (see Wischik *et al.* 2000, loc. cit, for detailed discussion - especially Table 5.1). All of these diseases, which are characterized primarily or partially by abnormal tau aggregation, are referred to herein as "tauopathies" or "diseases of tau protein aggregation".

In this and all other aspects of the invention relating to tauopathies, preferably the tauopathy is selected from the list consisting of the indications above, i.e., AD, Pick's disease, PSP, FTD, FTDP-17, DDPAC, PPND, Guam-ALS syndrome, PNLD, and CBD.

In one preferred embodiment the tauopathy is Alzheimer's disease (AD).

One aspect of the present invention pertains to a diaminophenothiazinium compound, as described herein, for use in a method of treatment or prophylaxis (e.g., of a tauopathy condition) of the human or animal body by therapy.

One aspect of the present invention pertains to use of a diaminophenothiazinium compound, as described herein, in the manufacture of a medicament for use in the treatment or prophylaxis of a tauopathy condition.

Also described herein is a method of treatment or prophylaxis of a disease of tau protein aggregation as described herein, which method comprises administering to a subject a diaminophenothiazinium compound, or therapeutic composition comprising the same, such as to inhibit the aggregation of the tau protein associated with said disease state.

### Other methods and uses

Also described herein is a diaminophenothiazinium compound, or therapeutic composition comprising the same, for use in a method of treatment or prophylaxis of a disease of tau protein aggregation as described above, which method comprises administering to a subject the diaminophenothiazinium compound or composition such as to inhibit the aggregation of the tau protein associated with said disease state.

Also described herein is use of a diaminophenothiazinium compound in the preparation of a medicament for use in a method of treatment or prophylaxis of a disease of tau protein aggregation as described above, which method comprises administering to a subject the medicament such as to inhibit the aggregation of the tau protein associated with said disease state.

Also described herein is a method of regulating the aggregation of a tau protein In the brain of a mammal, which aggregation is associated with a disease state as described above, the treatment comprising the step of administering to said mammal in need of said treatment, a prophylactically or therapeutically effective amount of an inhibitor of said aggregation, wherein the inhibitor is a diaminophenothiazinium compound.

Also described herein is a method of inhibiting production of protein aggregates (e.g. in the form of paired helical filaments (PHFs), optionally in neurofibrillary tangles (NFTs)) in the brain of a mammal, the treatment being as described herein.

Also described herein is a drug product for the treatment of a disease state associated with tau protein aggregation in a mammal suffering therefrom, comprising a container labeled or accompanied by a label indicating that the drug product is for the treatment of said disease, the container containing one or more dosage units each comprising at least one pharmaceutically acceptable excipient and, as an active ingredient, an isolated pure diaminophenothiazinium compound as described herein.

### Compositions, formulations and purity

Compositions and formulations are discussed in more detail hereinafter.

However, in one embodiment, the diaminophenothiazinium compound may be provided or used in a composition which is equal to or less than 100, 99, 98, 97, 96, 95, 94, 93, 92, 91, or 90% pure. In one embodiment the compound is not one which is obtained or obtainable by the high purity methods in disclosed PCT/GB2005/003634 (TauRx Therapeutics Pte. Ltd) which was filed, but not published, prior to the filing of the present application.

Also described herein is a dosage unit (e.g., a pharmaceutical tablet or capsule) comprising 20 to 300 mg of a diaminophenothiazinium compound as described herein (e.g., obtained by, or obtainable by, a method as described herein; having a purity as described herein; etc.).

Dosage units (e.g., a pharmaceutical tablet or capsule) comprising 20 to 300 mg of a diaminophenothiazinium compound as described herein and a pharmaceutically acceptable carrier, diluent, or excipient are discussed in more detail hereinafter.

In one embodiment, the amount is about 25 mg.

In one embodiment, the amount is about 35 mg.

In one embodiment, the amount is about 50 mg.

In one embodiment, the amount is about 70 mg.

In one embodiment, the amount is about 125 mg.

In one embodiment, the amount is about 175 mg.

In one embodiment, the amount is about 250 mg.

### Preferred dosage regimes

Dosage regimes are discussed in more detail hereinafter.

However in one embodiment, the diaminophenothiazinium compound is administered to a human patient according to the following dosage regime: about 50 or about 75 mg, 3 or 4 times daily.

In one embodiment, the diaminophenothiazinium compound is administered to a human patient according to the following dosage regime: about 100 or about 125 mg, 2 times daily.

### Preferred combination therapies

Combination treatments and therapies, in which two or more treatments or therapies are combined, for example, sequentially or simultaneously, are discussed in more detail hereinafter. Thus it will be understood that any of the medical uses or methods described herein may be used in a combination therapy.

In one embodiment, a treatment (e.g., employing a compound of the invention) is in combination with a cholinesterase inhibitor such as Donepezil (Aricept™), Rivastigmine (Exelon™) or Galantamine (Reminyl™).

In one embodiment, a treatment (e.g., employing a compound of the invention) is in combination with an NMDA receptor antagonist such as Memantine (Ebixa™, Namenda™).

In one embodiment, a treatment (e.g. employing a compound of the invention) is in combination with a muscarinic receptor agonist.

In one embodiment, a treatment (e.g. employing a compound of the invention) is in combination with an inhibitor of amyloid precursor protein to beta-amyloid (e.g., an inhibitor of amyloid precursor protein processing that leads to enhanced generation of beta-amyloid).

### Ligands and labels

Diaminophenothiazinium compounds discussed herein that are capable of inhibiting the aggregation of tau protein will also be capable of acting as ligands or labels of tau protein (or aggregated tau protein). Thus, in one embodiment, the diaminophenothiazinium compound is a ligand of tau protein (or aggregated tau protein).

Such diaminophenothiazinium compounds (ligands) may incorporate, be conjugated to, be chelated with, or otherwise be associated with, other chemical groups, such as stable and unstable detectable isotopes, radioisotopes, positron-emitting atoms, magnetic resonance labels, dyes, fluorescent markers, antigenic groups, therapeutic moieties, or any other moiety that may aid in a prognostic, diagnostic or therapeutic application.

For example, as noted above, in one embodiment, the diaminophenothiazinium compound is as defined above, but with the additional limitation that the compound incorporates, is conjugated to, is chelated with, or is otherwise associated with one or more (e.g., 1, 2, 3, 4, etc.) isotopes, radioisotopes, positron-emitting atoms, magnetic resonance labels, dyes, fluorescent markers, antigenic groups, or therapeutic moieties.

In one embodiment, the diaminophenothiazinium compound is a ligand as well as a label, e.g., a label for tau protein (or aggregated tau protein), and incorporates, is conjugated to, is chelated with, or is otherwise associated with, one or more (e.g., 1, 2, 3, 4, etc.) detectable labels.

For example, in one embodiment, the diaminophenothiazinium compound is as defined above, but with the additional limitation that the compound incorporates, is conjugated to, is chelated with, or is otherwise associated with, one or more (e.g., 1, 2, 3, 4, etc.) detectable labels.

Labelled diaminophenothiazinium compounds (e.g., when ligated to tau protein or aggregated tau protein) may be visualised or detected by any suitable means, and the skilled person will appreciate that any suitable detection means as is known in the art may be used.

For example, the diaminophenothiazinium compound (ligand-label) may be suitably detected by incorporating a positron-emitting atom (e.g., ¹¹C) (e.g., as a carbon atom of one or more alkyl group substituents, e.g., methyl group substituents) and detecting the compound using positron emission tomography (PET) as is known in the art.

Suitable methods for preparing these and similar ¹¹C labelled diaminophenothiaziniums are shown, for example, In WO 02/075318 (see Figures 11 a, 11 b, 12) and WO 2005/030676.

Described herein is a method of labelling tau protein (or aggregated tau protein) comprising the step of: contacting the tau protein (or aggregated tau protein) with a diaminophenothiazinium compound that incorporates, is conjugated to, is chelated with, or is otherwise associated with, one or more (e.g., 1, 2, 3, 4, etc.) detectable labels.

Also described herein is a method of detecting tau protein (or aggregated tau protein) comprising the steps of: contacting the tau protein (or aggregated tau protein) with a diaminophenothiazinium compound that incorporates, is conjugated to, is chelated with, or is otherwise associated with, one or more (e.g., 1, 2, 3, 4; etc.) detectable labels, and detecting the presence and\or amount of said compound bound to tau protein (or aggregated tau protein).

Also described herein is a method of diagnosis or prognosis of a tau proteinopathy in a subject believed to suffer from the disease, comprising the steps of:
(i) introducing into the subject a diaminophenothiazinium compound capable of labelling tau protein or aggregated tau protein, particularly tau protein (e.g., a diaminophenothiazinium compound that incorporates, is conjugated to, is chelated with, or is otherwise associated with, one or more (e.g., 1, 2, 3, 4, etc.) detectable labels),
(ii) determining the presence and\or amount of said compound bound to tau protein or aggregated tau protein in the brain of the subject,
(iii) correlating the result of the determination made in (ii) with the disease state of the subject.

Also described herein is a diaminophenothiazinium compound capable of labelling tau protein or aggregated tau protein (e.g., a diaminophenothiazinium compound that incorporates, is conjugated to, is chelated with, or is otherwise associated with, one or more (e.g., 1,2, 3,4, etc.) detectable labels), for use in a method of diagnosis or prognosis of a tau proteinopathy.

Also described herein is use of a diaminophenothiazinium compound capable of labelling tau protein or aggregated tau protein, particularly tau protein (e.g., a diaminophenothiazinium compound that incorporates, is conjugated to, is chelated with, or is otherwise associated with, one or more (e.g., 1, 2, 3,4, etc.) detectable labels), in a method of manufacture of a diagnostic or prognostic reagent for use in the diagnosis or prognosis of a tau proteinopathy.

Those skilled in the art will appreciate that instead of administering diaminophenothiazinium ligands/labels directly, they could be administered in a precursor form, for conversion to the active form (e.g., ligating form, labelling form) by an activating agent present in, or administered to, the same subject.

The ligands disclosed herein may be used as part of a method of diagnosis or prognosis. It may be used to select a patient for treatment, or to assess the effectiveness of a treatment or a therapeutic (e.g. an inhibitor of tau protein aggregation) administered to the subject.

### Methods of Synthesis

Methods for the chemical synthesis of compounds of the present invention are described in the Examples herein. These and/or other well known methods may be modified and/or adapted in known ways in order to facilitate the synthesis of other compounds of the present invention.

Described herein is a method of synthesising a compound of the invention as described herein, described, or substantially as described, with reference to any of the Examples hereinafter.

Also described herein is a diaminophenothiazinium compound of the invention which is *obtained by or is obtainable by,* a method as described herein.

Some aspects will now be explained in more detail:

### Treatment

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, regression of the condition, amelioration of the condition, and cure of the condition. Treatment as a prophylactic measure (i.e., prophylaxis, prevention) is also included.

The term "therapeutically-effective amount," as used herein, pertains to that amount of an active compound, or a material, composition or dosage from comprising an active compound, which is effective for producing some desired therapeutic effect, commensurate with a reasonable benefit/risk ratio, when administered in accordance with a desired treatment regimen.

Similarly, the term "prophylactically-effective amount," as used herein, pertains to that amount of an active compound, or a material, composition or dosage from comprising an active compound, which is effective for producing some desired prophylactic effect, commensurate with a reasonable benefit/risk ratio, when administered in accordance with a desired treatment regimen.

The term "treatment" includes combination treatments and therapies, in which two or more treatments or therapies are combined, for example, sequentially or simultaneously. Examples of treatments and therapies include, but are not limited to, chemotherapy (the administration of active agents, including, e.g., drugs, antibodies (e.g., as in immunotherapy), prodrugs (e.g., as in photodynamic therapy, GDEPT, ADEPT, etc.)); surgery; radiation therapy; and gene therapy.

### Routes of Administration

The diaminophenothiazinium compound, or pharmaceutical composition comprising it, may be administered to a subject/patient by any convenient route of administration, whether systemically/peripherally or topically (i.e., at the site of desired action).

Routes of administration include, but are not limited to, oral (e.g., by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eyedrops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal (including, e.g., intracatheter injection into the brain); by implant of a depot or reservoir, for example, subcutaneously or intramuscularly.

### The Subject/Patient

The subject/patient may be an animal, mammal, a placental mammal, a marsupial (e.g., kangaroo, wombat), a monotreme (e.g., duckbilled platypus), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orangutang, gibbon), or a human.

Furthermore, the subject/patient may be any of its forms of development, for example, a foetus.

In one preferred embodiment, the subject/patient is a human.

Suitable subjects for the method may be selected on the basis of conventional factors. Thus the initial selection of a patient may involve any one or more of: rigorous evaluation by experienced clinician; exclusion of non-AD diagnosis as far as possible by supplementary laboratory and other investigations; objective evaluation of level of cognitive function using neuropathologically validated battery.

In one embodiment, the subject/patient is not a human.

### Formulations

While it is possible for the diaminophenothiazinium compound to be used (e.g., administered) alone, it is often preferable to present it as a composition or formulation.

In one embodiment, the composition is a pharmaceutical composition (e.g., formulation, preparation, medicament) comprising a diaminophenothiazinium compound, as described herein, and a pharmaceutically acceptable carrier, diluent, or excipient.

In one embodiment, the composition is a pharmaceutical composition comprising at least one diaminophenothiazinium compound, as described herein, together with one or more other pharmaceutically acceptable ingredients well known to those skilled in the art, including, but not limited to, pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents.

In one embodiment, the composition further comprises other active agents, for example, other therapeutic or prophylactic agents.

Suitable carriers, diluents, excipients, etc. can be found in standard pharmaceutical texts. See, for example, Handbook of Pharmaceutical Additives, 2nd Edition (eds. M. Ash and I. Ash), 2001 (Synapse Information Resources, Inc., Endicott, New York, USA), Remington's Pharmaceutical Sciences, 20th edition, pub. Lippincott, Williams & Wilkins, 2000; and Handbook of Pharmaceutical Excipients, 2nd edition, 1994.

Also described herein are methods of making a pharmaceutical composition comprising admixing at least one [¹¹C]-radiolabelied phenothiazine or phenothiazine-like compound, as defined herein, together with one or more other pharmaceutically acceptable ingredients well known to those skilled in the art, e.g., carriers, diluents, excipients, etc. If formulated as discrete units (e.g., tablets, etc.), each unit contains a predetermined amount (dosage) of the active compound.

The term "pharmaceutically acceptable," as used herein, pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation.

The formulations may be prepared by any methods well known in the art of pharmacy. Such methods include the step of bringing into association the active compound with a carrier which constitutes one or more accessory ingredients. In general, the formulations are prepared by uniformly and intimately bringing into association the active compound with carriers (e.g., liquid carriers, finely divided solid carrier, etc.), and then shaping the product, if necessary.

The formulation may be prepared to provide for rapid or slow release; immediate, delayed, timed, or sustained release; or a combination thereof.

Formulations suitable for parenteral administration (e.g., by injection), include aqueous or non-aqueous, isotonic, pyrogen-free, sterile liquids (e.g., solutions, suspensions), in which the active ingredient Is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. Typically, the concentration of the active ingredient in the liquid is from about 1 ng/ml to about 10 µg/ml, for example from about 10 ng/ml to about 1 µg/ml. The formulations may be presented In unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

### Examples of Formulations

Also described herein is a dosage unit (e.g., a pharmaceutical tablet or capsule) comprising 20 to 300 mg of a diaminophenothiazinium compound as described herein (e.g., obtained by, or obtainable by, a method as described herein; having a purity as described herein; etc.), and a pharmaceutically acceptable carrier, diluent, or excipient.

In one embodiment, the dosage unit is a tablet.

In one embodiment, the dosage unit is a capsule.

In one embodiment, the amount is 30 to 200 mg.

In one embodiment, the amount is about 30 mg.

In one embodiment, the amount is about 60 mg.

In one embodiment, the amount is about 100 mg.

In one embodiment, the amount is about 150 mg.

In one embodiment, the amount is about 200 mg.

In one embodiment, the pharmaceutically acceptable carrier, diluent, or excipient is or comprises one or both of a glyceride (e.g., Gelucire 44/14 ®; lauroyl macrogol-32 glycerides PhEur, USP) and colloidal silicon dioxide (e.g., 2% Aerosil 200 ®; Colliodal Silicon Dioxide PhEur, USP).

### Dosage

It will be appreciated by one of skill in the art that appropriate dosages of the diaminophenothiazinium compound, and compositions comprising the diaminophenothiazinium compound, can vary from patient to patient. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the patient. The amount of compound and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of the active compound is in the range of about 100 ng to about 25 mg (more typically about 1 µg to about 10 mg) per kilogram body weight of the subject per day. Where the active compound is a salt, an ester, an amide, a prodrug, or the like, the amount administered is calculated on the basis of the parent compound and so the actual weight to be used is increased proportionately.

In one embodiment, the active compound is administered to a human patient according to the following dosage regime: about 100 mg, 3 times daily.

In one embodiment, the active compound is administered to a human patient according to the following dosage regime: about 150 mg, 2 times daily.

In one embodiment, the active compound is administered to a human patient according to the following dosage regime: about 200 mg, 2 times daily.

The invention will now be further described with reference to the following non-limiting Examples. Other embodiments of the invention will occur to those skilled in the art in the light of these.

### EXAMPLES

### Example 1 - Methods of Synthesis

The following syntheses are provided solely for illustrative purposes.

### Synthesis 1

### Ethyl-thioninium chloride (ETC)

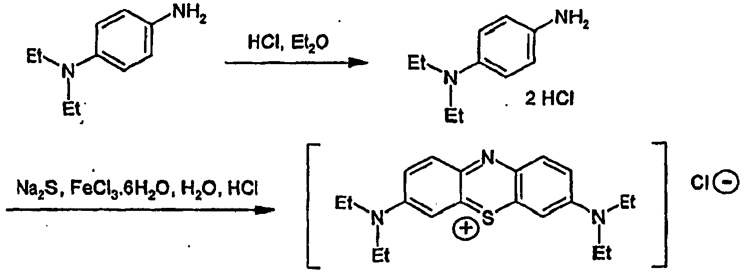

### N,N-diethyl-p-phenylenediamine dihydrochloride

*N,N*-diethyl-*p*-phenylenediamine (5 g, 30.4 mmol) was dissolved in diethyl ether (25 cm³) and hydrochloric acid (6 cm³, 10 M) was added and the mixture was concentrated to give the title compound (7.22 g, 100%) as a red/brown solid. δ_{H} (250 MHz; D₂O): 7.68 (4H, m, ArH), 3.69 (4H, q, 7.32, NCH₂), 1.11 (6H, t, 7.32, CH₃); δ_{C} (62.9 MHz; D₂O): 12.1 (CH₃), 56.4 (NCH₂), 126.8 (ArC), 127.6 (ArC), 135.5 (ArC), 139.1 (ArC).

### Ethyl-thioninium chloride

*N,N*-diethyl-*p*-phenylenediamine dihydrochloride (7.22 g, 30.4 mmol) was dissolved in water (250 cm³) and the pH adjusted to 1.6 with HCl, to which sodium sulphide (>60%) (3.95 g, 30.4 mmol) was added portionwise. The suspension was stirred until all the sodium sulphide had dissolved. A solution of iron (III) chloride (27.15 g, 100 mmol) in water (200 cm³) was prepared and half the solution was added to the mixture. An immediate colour change from light yellow to blue occurred. The solution was then aerated for 1 hour before the remaining iron (III) chloride solution was added. The mixture was cooled to 5°C and filtered to remove a light green sludge. Aqueous HCl (15 cm³, 6 M) was added to the filtrate, followed by sodium chloride (60 g), and the suspension stirred for 5 minutes before filtering to give a solid product, which was dissolved in DCM, dried over magnesium sulphate, filtered, and concentrated to give a purple/green solid (1.28 g, 22%). This purple/green solid was loaded onto a prepared C18 reverse phase column and washed with water (1 L) or until the yellow colour ceased. The product was washed off the column with MeOH/HCl (pH 2) and concentrated to give the title compound (0.64 g, 11%) as a sticky purple solid. δ_{H} (250 MHz; D₂O): 1.26(12H, t, 6.5, CH₃), 3.56 (8H, q, 6.5, NCH₂), 7.01 (2H, s, ArH), 7.20 (2H, d, 9.25, ArH), 7.54 (2H, d, 9.25, ArH); m/z (ESI) 340.2 (100%, [M-CI]⁺).

### (Reference) Synthesis 2

### 1,9-Dimethyl-methyl-thioninium chloride (DMMTC)

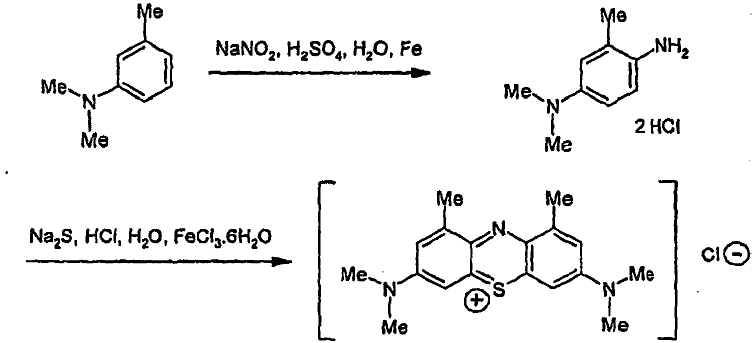

### 3-Methyl-N,N-dimethylphenylenediamine dihydrochloride

To a 250cm³ round bottom flask was added water (100 cm³) and the temperature was reduced to 5°C with an ice bath. To this cooled solution was carefully added sulphuric acid (98%, 22.5 g). To this solution was added 3-methyl-*N,N*-dimethylaniline (10 g, 74 mmol) and then sodium nitrite (5.6 g, 81.4 mmol), and the solution was stirred at room temperature for 1 hour. Iron (Fe) filings (12.8 g, 229 mmol) were added and the mixture stirred for a further 2 hours. The solution was filtered and then neutralized with saturated sodium hydrogen carbonate solution and the organics were extracted into ethyl acetate (3 x 100 cm³). The extracts were dried over magnesium sulphate, filtered, and concentrated to give a brown oil. The oil was dissolved in diethyl ether (100 cm³) and concentrated hydrochloric acid (50 cm³) was added. The solution was evaporated to dryness to give the title compound (10 g, 60%) as a light tan solid. νₘₐₓ (KBr)/cm⁻¹: 2849 (*CH*), 2821 (*CH*), 2543 (*CH*), 2444 (*CH*), 1586 (*C*=*N*), 1487 (*CH*), 1445 (*CH*), 1415 (*CH*), 1138 (*CH*); δ_{H} (250 MHz; D₂O): 7.59 (1H, s, ArH), 7.50 (2H, s, ArH), 3.24 (6H, s, CH₃), 2.39 (3H, s, CH₃); δ_{C}(62.9 MHz; D₂O) 18.9 (CH₃), 48.8 (CH₃), 122.1 (ArC), 126.2 (ArC), 127.6 (ArC), 133.7 (ArC), 137.4 (ArC), 144.4 (ArC).

### Dimethylmethythioninium Chloride

To a 500 cm³ round bottom flask was added 3-methyl-*N,N*-dimethyl-phenylene-diamine dihydrochloride (0.9 g, 4.03 mmol) which was dissolved in aqueous hydrochloric acid (50 cm³, 3 M) before sodium sulphide (>60%) (0.52 g, 4.03 mmol) was added. Iron (III) chloride hexahydrate (7.26 g, 27 mmol) was dissolved in water (50 cm³) and half of this solution was poured into the reaction mixture, giving an immediate blue colour. The solution was then aerated for 2 hours before the remaining aqueous iron (III) chloride solution was added. The mixture was cooled to 5°C and filtered; the precipitate was dissolved in boiling water (60 cm³), filtered, and cooled. Hydrochloric acid (10 cm³, 6 M) was added to the cooled solution, which was then filtered to yield the title compound (0.22 g, 16%) as a purple/blue solid. νₘₐₓ (KBr)/cm⁻¹: 2926 (*CH*), 1604 (*C*=*N*), 1535, 1496, 1444 (*CH*), 1404 (*CH*), 1315 (*CH*), 1185 (*CH*); δ_{H} (250 MHz; DMSO): 7.29 (2H, s, ArH), 7.23 (2H, s, ArH), 3.29 (12H, s, CH₃), 2.55 (6H, s, CH₃); δ_{C} (62.9 MHz; DMSO): 18.9 (CH₃), 41.5 (CH₃), 105.7 (ArC), 118.7 (ArC), 133.6 (ArC), 134.5 (ArC), 147.2 (ArC), 154.2 (ArC); Anal. Calcd. for C₁₈H₂₂N₃S.3H₂O: C, 51.98; H, 6.74; N, 10.11; S, 7.70. Found: C, 52.03; H, 6.59; N, 10.05; S, 7.66.

### Synthesis 3

### 1,9-Diethyl-methyl-thioninium chloride (DEMTC)

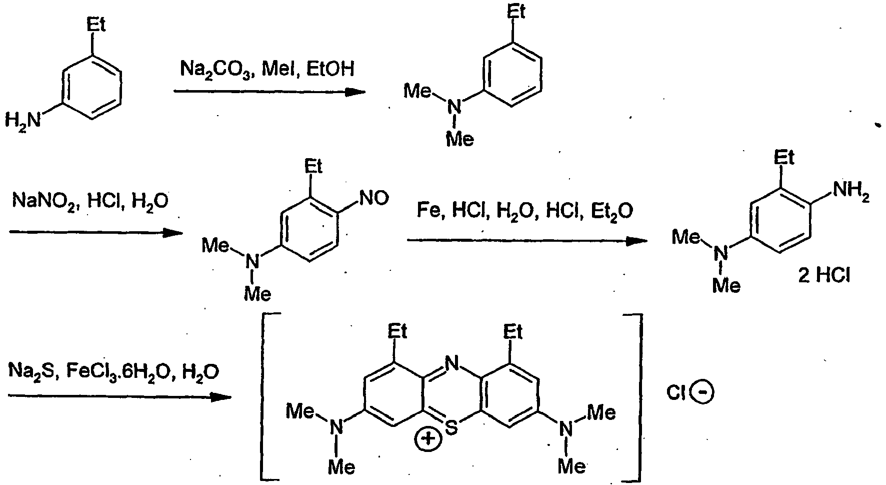

### N,N-Dimethyl-m-ethylaniline

To a 100 cm³ round bottom flask was added 3-ethylaniline (10 g, 82.5 mmol), ethanol (15 cm³), sodium carbonate (11.81 g, 111.4 mmol). Methyl iodide (31.63 g, 222 mmol) was added dropwise. The mixture was then heated at 45°C for 10 hours before cooling to room temperature and adding water (100 cm³). The mixture was extracted into diethyl ether (3 x 100 cm³) and the extracts were dried over magnesium sulphate, filtered, and concentrated to give the title compound (4.68 g, 38%) as a light yellow oil. νₘₐₓ (neat)/cm⁻¹: 3045 (*CH*), 2960 (*CH*), 2920 (*CH*), 2891 (*CH*), 2797 (*CH*), 1597 (*C*=*N*), 1494 (*CH*), 1438 (*CH*), 1352 (*CH*), 1225 (*CH*); δ_{H} (250 MHz; CDCl₃): 7.22 (1H, t, 7.75, ArH), 6.63 (3H, m, ArH), 2.97 (6H, s, NCH₃), 2.63 (2H, q, 7.5, CH₂), 1.27 (3H, t, 7.5, CH₃); δ_{C} (62.9 MHz; CDCl₃): 15.8 (CH₃), 29.5 (NCH₂), 40.8 (NCH₃), 110.3 (ArC), 112.4 (ArC), 116.5 (ArC), 129.1 (ArC), 145.3 (ArC), 150.9 (ArC).

### N,N-Dimethyl-m-ethyl-p-phenylenediamine dihydrochloride

To a 250 cm³ round bottom flask was added *N,N*-dimethyl-*m*-ethylaniline (4.68 g, 31.3 mmol), water (100 cm³) and hydrochloric acid (8.5 cm³, 37%) and the solution was cooled to 5°C. An aqueous (80 cm³) solution of sodium nitrite (2.46 g, 3.57 mmol) was then added dropwise to the aniline mixture and stirred for 3 hours at room temperature. Iron (Fe) fillings (5.24 g, 94 mmol) and hydrochloric acid (8.5 cm³, 37%) were added and the mixture was stirred at room temperature for 3 hours. The suspension was filtered and the filtrate adjusted to pH 7 with sodium bicarbonate solution before extraction into ethyl acetate (3 x 50 cm³). The combined extracts were dried over magnesium sulphate, filtered, and concentrated to yield a brown oil. The oil was dissolved in ethanol (100 cm³) and diethyl ether (80 cm³) and hydrochloric acid (7cm³, 37%) was added carefully to give the title compound (7.42 g, 72%) as a light tan solid. νₘₐₓ (KBr)/cm⁻¹: 2976 (*CH*), 2894 (*CH*), 2859 (*CH*), 2753 (*CH*), 1583 (*C*=*N*), 1508 (*CH*), 1486 (*CH*), 1459 (*CH*), 1183 (*CH*); δ_{H} (250 MHz; D₂O): 7.66 (1H, s, ArH), 7.56 (2H, s, ArH), 3.29 (6H, s, NCH₃), 2.74 (2H, q, 7.5, CH₂), 1.25 (3H, t, 7.5, CH₃); δ_{C} (62.9 MHz; CDCl₃): 15.5 (CH₃) 25.6 (NCH₂), 48.9 (NCH₃), 122.1 (ArC), 124.6 (ArC), 128.1 (ArC), 132.6 (ArC), 143.3 (ArC), 144.9 (ArC).

### 1,9-Diethyl Methylthioninium chloride

*N,N*-Dimethyl-*m*-ethyl-p-phenylenediamine dihydrochloride (1.3 g, 5.5 mmol) was dissolved in water (50 cm³) and the solution adjusted to pH 1.6. Sodium sulphide >60% (0.71g, 5.5mmol) was then added portionwise to the pink solution. To the suspension was added an aqueous solution of iron (III) chloride (2.23 g, 8.2 mmol in 50 cm³ of water) and there was an immediate colour change to purple. The solution was then aerated for 1 hour before a second portion of iron (III) chloride solution (2.23 g, 8.2 mmol in 50 cm³ of water) was added. The solution was cooled to 5°C before filtering and washing the precipitate with water. To the filtrate was added sodium chloride (50 g) and the solution was stirred for 10 minutes, and the colour changed to red/purple as the product was salted out. The suspension was filtered and the solid dissolved in dichloromethane (100 cm³) and methanol (10 cm³) before drying over magnesium sulphate. Filtration and concentration gave the title compound (0.15 g, 15%) as a green solid. νₘₐₓ (KBr)/cm⁻¹: 3408 (*CH*), 2613 (*CH*), 1606 (C=*N*), 1399 (*CH*), 1316 (*CH*); δ_{H} (250 MHz; D₂O): 6.55 (2H, s, ArH), 6.23 (2H, s, ArH), 2.92 (12H, s, NCH₃), 2.56 (4H, q, 7.5, CH₂), 0.99 (6H, t, 7.5, CH₃); (ESI), 340.4 (100%, [M - Cl]⁺). Optionally, flash column chromatography was performed to remove iron chloride residues, with 10% methanol: 90% dichloromethane as eluent and using silica 40-63µ 60Ǻ.

### Synthesis 4

### 1,9-Dimethyl-ethyl-thioninium chloride (DMETC)

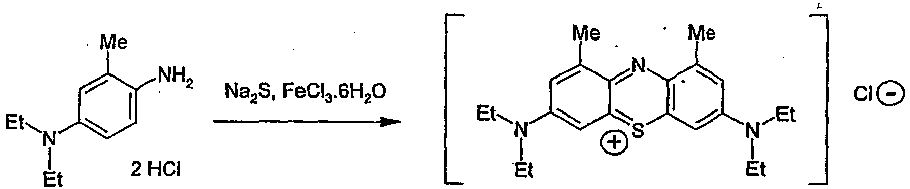

*N,N*-Diethyl-3-methyl-4-phenylenediamine dihydrochloride (10.74 g, 50 mmol) was dissolved in water (400 cm³) and the pH adjusted to 1.6, which then had sodium sulphide (>60%) (3.90 g, 50 mmol) added. Iron (III) chloride (20.28 g, 75 mmol) was added as an aqueous solution (175 cm³) giving an immediate colour change from yellow to deep blue. The mixture was aerated for 1 hour before a second aliquot of aqueous iron (III) chloride (20.28 g, 75 mmol in 175 cm³) was added. The solution was cooled to 5°C and held at that temperature for 1 hour before filtering. The filtrate had sodium chloride (200 g) added and was filtered to yield the crude product as a blue/purple solid. The crude solid was purified by column chromatography (eluent being 10% MeOH, 90% DCM using silica 40-63µ 60Ǻ) to give the title compound (0.80 g, 4%) as a green/purple solid. νₘₐₓ (KBr)/cm⁻¹: 2971 (*CH*), 2921 (*CH*), 2865 (*CH*), 1600 *(C=N),* 1412 (*CH*), 1326 (*CH*); δ_{H} (250 MHz; D₂O): 6.62 (2H, s, ArH), 6.39 (2H, s, ArH), 3.30 (8H, q, NCH₂), 1.89 (6H, s, ArCH₃), 1.09 (12H, t, CH₃); δ_{C} (62.9 MHz; D₂O) 12.6 (CH₃), 18.0 (CH₃), 46.2 (NCH₂), 103.6 (ArC), 117.1 (ArC), 132.3 (ArC), 133.9 (ArC), 147.3 (ArC), 151.9 (ArC); m/z (ESI) 368.1 (100%, [M-Cl]⁺).

### Synthesis 5

### 1,9-Diethyl-ethyl-thioninium chloride (DEETC)

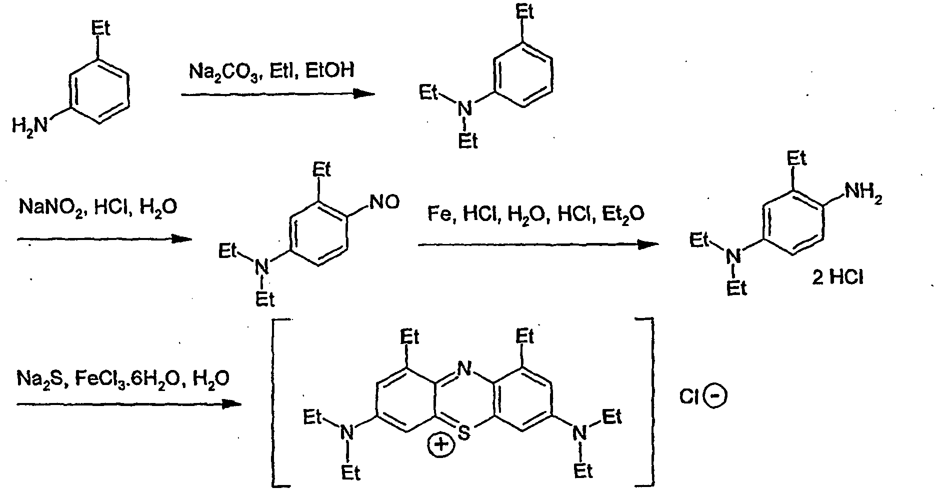

### N,N-Diethyl-m-ethylaniline

To a 100 cm³ round bottom flask was added 3-ethylaniline (5.0 g, 41.3 mmol), ethanol (7.5 cm³), sodium carbonate (5.9 g, 55.7 mmol). Ethyl iodide (17.38 g, 111.4 mmol) was added dropwise. The mixture was then heated at 45°C for 12 hours before cooling to room temperature and adding water (50cm³). The mixture was extracted into diethyl ether (3 x 50 cm³) the extracts were dried over magnesium sulphate, filtered, and concentrated to give the title compound (7.03 g, 96%) as a light yellow oil. δ_{H} (250 MHz; CDCl₃): 7.20 (1 H, dd, 9, 7.25, ArH), 6.60 (3H, m, ArH), 3.43 (4H, q, 7, NCH₂), 2.69 (2H, q, 7.25, CH₂), 1.32 (3H, t, 7.5, CH₃), 1.23 (6H, t, 7, CH₃); δ_{C} (62.9 MHz; CDCl₃): 12.7 (CH₃), 15.8 (CH₃), 29.5 (CH₂), 44.4 (NCH₃), 109.4 (ArC), 111.4 (ArC), 115.1 (ArC), 129.2 (ArC), 145.4 (ArC), 147.9 (ArC).

### N,N-Diethyl-m-ethyl-p-phenylenediamine dihydrochloride

To a 250 cm³ round bottom flask was added *N,N*-diethyl-*m*-ethy(aniline (5 g, 28.2 mmol), water (50 cm³) and hydrochloric acid (9 cm³, 37%) and the solution was cooled to 5°C. An aqueous (20 cm³) solution of sodium nitrite (2.14 g, 31.0 mmol) was then added dropwise to the aniline mixture and stirred for 1 hour at low temperature. Iron (Fe) fillings (4.72 g, 84.6 mmol) and hydrochloric acid (9 cm³, 37%) were added and the mixture stirred below 30°C for 2 hours. The suspension was filtered and the filtrate adjusted to pH 7 with sodium bicarbonate solution before extraction into ethyl acetate (3 x 50 cm³). The combined extracts were dried over magnesium sulphate, filtered, and concentrated to yield a brown oil. The crude oil was purified by column chromatography (eluent being ethyl acetate using silica 40-63µ 60A) giving the phenylenediamine as a brown oil (2.2 g, 41%). The oil was dissolved in diethyl ether (50 cm³) and hydrochloric acid added (2.5cm³, 37%) and the solution was concentrated to give the title compound (2.76 g, 41%) as a light brown solid. δ_{H} (250 MHz; D₂O): 7.50 (3H, m, ArH), 3.59 (4H, q, 7.25, NCH₂), 2.69 (2H, q, 7.5, CH₂), 1.20 (3H, t, 7.5, CH₃), 1.03 (6H, t, 7.25, CH₃); δ_{C} (62.9 MHz; D₂O): 12.1 (CH₃), 15.5 (CH₃), 25.5 (CH₂), 56.3 (NCH₂), 123.9 (ArC), 126.0 (ArC), 127.9 (ArC), 133.1 (ArC), 139.4 (ArC), 143.3 (ArC).

### 1,9-Diethyl Ethyl-thioninium chloride

*N,N*-Diethyl-*m*-ethyl-*p*-phenylenediamine dihydrochloride (2 g, 7.5 mmol) was dissolved in water (75 cm³) and the solution adjusted to pH 1.6. The pink solution then had sodium sulphide (>60%) (1.35g, 10.4mmol) added portion-wise. To the suspension was added an aqueous solution of iron (III) chloride (4.22 g, 15.6 mmol in 35 cm³ of water) where there was an immediate colour change to purple. The solution was then aerated for 1 hour before a second portion of iron (III) chloride (4.22 g, 15.6 mmol in 35 cm³ of water) solution was added. The solution was cooled to 5°C before filtering and washing the precipitate with water. The precipitate was also washed with ethanol and the ethanol concentrated to give a sticky purple solid. To the aqueous filtrate was added sodium chloride (50 g) and the solution was stirred for 10 minutes whereby the colour changed to red/purple as the product was salted out. The suspension was filtered and the solid dissolved in dichloromethane (100 cm³) and methanol (10 cm³) before drying over magnesium sulphate. Filtering and concentration with the ethanol soluble product gave the title compound (0.06 g, 3%) as a purple solid. δ_{H} (250 MHz; D₂O): 6.73 (2H, s, ArH), 6.48 (2H, s, ArH), 3.45 (8H, brdq, NCH₂), 2.46 (4H, q, 7.5, CH₂), 1.17 (12H, brdt, CH₃), 0.93 (6H, t, 7.5, CH₃); *m*/*z* (ESI) 396.2 (100%, [M-Cl]⁺). Optionally, flash column chromatography was performed to remove iron chloride residues, with 10% methanol: 90% dichloromethane as eluent and using silica 40-63µ 60A.

### Synthesis 6

### Ethyl-thioninium chloride zinc chloride double salt (ETZ)

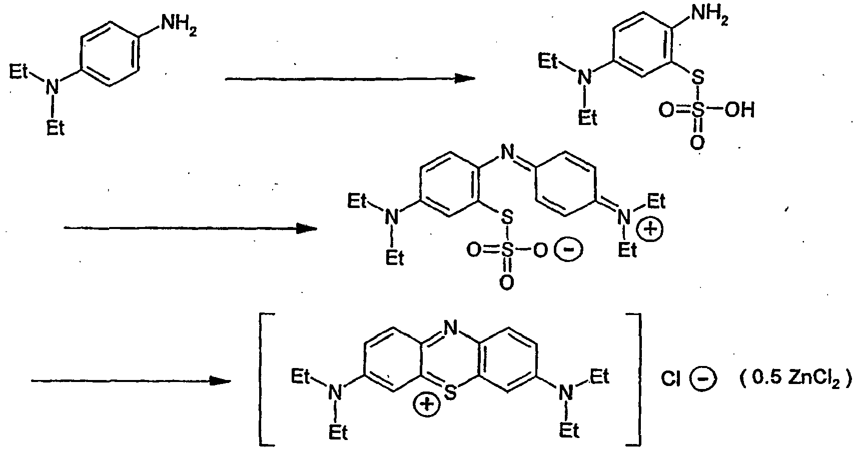

A stirred mixture of *N*,*N*-diethyl-*p*-phenylenediamine (5.0 g, 30.4 mmol) in *H₂*O (100 cm³) and H₂SO₄ (conc., '98 %', 1 cm³) was treated with non-reducing ZnCl₂ solution (ZnCl₂, 7.60 g, 55 mmol in 15 cm³ of H₂O with Na₂Cr₂O₇.2H₂O, 100 mg) to produce a reddish reaction mixture. Additions of Al₂(SO₄)₃.16H₂O solution (5.80 g, 9.2 mmol in 10 cm³ of H₂O), N₃₂S₂O₃.5H₂O solution (8.0 g, 32.2 mmol in 10 cm³ H₂O) and one-third of a solution of Na₂Cr₂O₇.2H₂O (8.7 g, 29.2 mmol in 15 cm³ of H₂O) were followed by a rapid rise in temperature to 40°C. A solution of *N*,*N*-diethylaniline (3.0 g, 20.1 mmol in conc. HCl, 4 cm³) was added, and followed by an addition of the remaining Na₂Cr₂O₇.2H₂O solution. A dark green precipitate was observed. The temperature was rapidly raised to 75°C, after which a slurry of activated MnO₂ (3.80 g, 44.7 mmol in 5 cm³ of H₂O) was added. The temperature was raised to 85°C, and left to stir at that temperature for 30 minutes. A blue solution with precipitate was observed. The reaction mixture was cooled to 50°C and H₂SO₄ (conc., 11cm³) was slowly added. The reaction was further cooled to 20°C, and vacuum filtered to recover the precipitate, which was then washed with brine (saturated salt water). This black solid was re-dissolved in H₂O (250 cm³) at 100°C, and cooled, followed by vacuum filtration to remove insolubles. The filtrate was treated with ZnCl₂ (4 g) and NaCl (23 g) and left in the refrigerator for 16 hours, after which the resulting precipitate was recovered by vacuum filtration, washed with brine (30 cm³), and dried in a vacuum oven for 3 hours, to give the title compound (5.7 g, 71 %) as a rusty red powder. δ_{H} (250 MHz, D₂O): 1.20 (12H, br t, CH₃), 3.50 (8H, br q, CH₂), 6.80 (2H, s, ArH), 7.05 (2H, br d, ArH) and 7.30 (2H, br d, ArH). See, for example, Fierz-David and Blangley, 1949, "F. Oxazine and Thiazine Dyes," in: Fundamental Processes of Dye Chemistry, published by Interscience (London, UK), pp. 308-314.

### (Reference) Synthesis 7

### Methyl-thioninium Iodide (MTI)

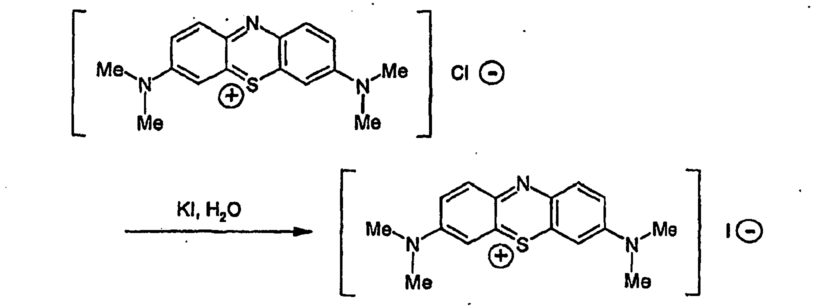

Methyl-thioninium chloride (2.00 g, 6.25 mmol) was dissolved in water (50 cm³) and potassium iodide (1.56 g, 9.4 mmol) was added with stirring. A precipitate formed, which was filtered and the solid was recrystallised from boiling water (50 cm³) to yield the title compound (1.98 g, 77%) as fine green needles. δ_{H} (250 MHz; DMSO): 7.88 (2H, br d, Ar*H*), 7.49 (4H, br s, Ar*H*), 3.37 (12H, s, CH₃). Analysis for C₁₆H₁₈N₃Sl: C, 46.72; H, 4.41; N, 10.22; S, 7.80; I, 30,85; Found: C, 46.30; H, 4.21; N, 10.14; S, 7.86; I, 29.34.

### (Reference) Synthesis 8

### Methyl-thioninium Iodide Hydrogen Iodide Mixed Salt (MTI.HI)

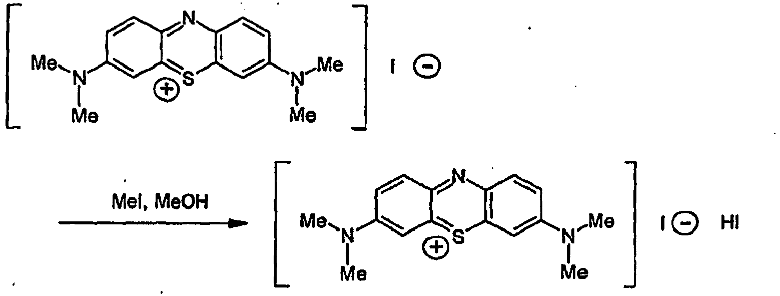

Methyl-thioninium iodide (0.50 g, 1.22 mmol) was dissolved in methanol (20 cm³) and methyl iodide (1.90 g, 13.37 mmol) was added while stirring. The mixture was heated at reflux for 18 hours before additional methyl iodide (0.42 g, 6.69 mmol) was added and the mixture was once again heated to reflux and stirred for 8 hours. The mixture was cooled to room temperature, giving a solid that was filtered and washed with methanol to yield the title compound (0.30 g, 46%) as bronze green solid. δ_{H} (250 MHz; DMSO): 7.82 (2H, d, *J* = 8.5, Ar*H*), 7.42 (4H, s, Ar*H*), 3.34 (12H, s, CH₃). δ_{c} (62.9 MHz; DMSO): 153.8 (ArC), 137.9 (ArC), 134.9 (ArC), 133.5 (Ar*C*), 119.1 (ArC), 118.8 (ArC), 106.9 (Ar*C*), 106.6 (ArC), 41.1 (N*C*H₃).

### Synthesis 9

### Ethyl-thioninium iodide (ETI)

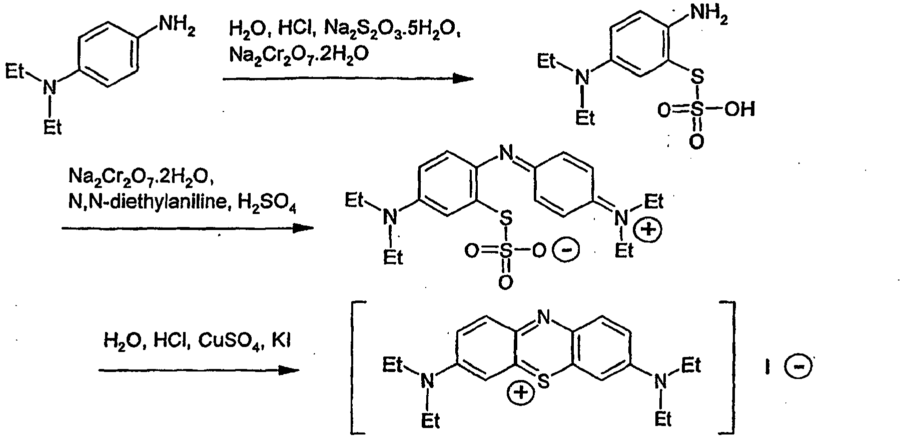

A stirred mixture of *N*,*N*-diethyl-p-phenylenediamine (10.0 g, 61 mmol) in aqueous hydrochloric acid (0.5 M, 200 cm³) was adjusted to pH 2 with aqueous sodium hydroxide (10%). The diamine solution was cooled to 5°C before the addition of aqueous Na₂S₂O₃.5H₂O (16.65 g, 67 mmol in 20 cm³ H₂O). An aqueous solution of Na₂Cr₂O₇.2H₂O (7.27 g, 24 mmol in 35 cm³ of H₂O) was added dropwise to the mixture over a 15 minute period giving a black suspension. The suspension was stirred at 5°C for 1 hour (pH = 8.07, T = 3.7°C). A solution of *N*,*N*-diethylaniline (8.25 g, 61 mmol), H₂SO₄ (6 g) and water (10 cm³) was cooled to 5°C before addition to the suspension. An aqueous solution of Na₂Cr₂O₇.2H₂O (19.09 g, 64 mmol in 50 cm³ of H₂O) was then added dropwise to the mixture over a 20 minute period giving a thick dark green suspension. The mixture was stirred at 5°C for 2 hours (pH = 6.75, T = 6°C) before filtering. The green purple solid obtained was washed with water (2 x 50 cm³). The solid was slurried in aqueous hydrochloric acid (300 cm³, pH 2) giving a suspension with a pH = 6.37 at 22°C. To the suspension was added CuSO₄ (1.52 g, 6.1 mmol) and the mixture heated to 90°C where a deep blue solution formed. After stirring at this temperature for 1 hour the mixture was cooled to 25°C and filtered. The solid was washed with water (2 x 50 cm³), the filtrate was adjusted from pH 6.33 to pH 2.00, T = 25°C with hydrochloric acid (5 M). The deep blue solution was heated to 80°C and potassium iodide (14 g) was added and upon cooling an orange purple precipitate was deposited. Filtration gave a purple powder (8.8 g, 31%), which was recrystallised from hot ethanol (400 cm³) to give the title compound as fine purple needles. Mp 211°C; vₘₐₓ (KBr)/cm⁻¹: 3574 (*CH*), 3484 (*CH*), 3028 (*CH*), 2965 (*CH*), 1662 (*C*=*C*), 1539 (*CH*), 1474 (*CH*), 1346 (*CH*); δ_{c} (62.9 MHz, CDCl₃): 1.33 (12H, t, 7, CH₃), 3.72 (8H, q, 7, NCH₂), 7.23 (2H, d, 9.75, ArH), 7.41 (2H, s, ArH), 7.83 (2H, d, 9.75, ArH); δ_{H} (62.9 MHz, CDCl₃):152.4, 138.8, 135.7, 135.2, 118.3, 106.4, 46.8, 13.2.

### Synthesis 10

### Ethyl-thioninium iodide Hydrogen Iodide Mixed Salt (ETI.HI)

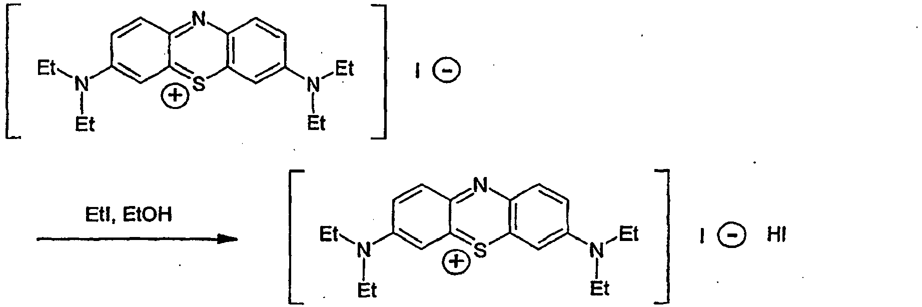

Ethyl-thioninium iodide (2.00 g, 4.28 mmol) was dissolved in ethanol (100 cm³) and ethyl iodide (27.35 g, 175 mmol) was added while stirring. The mixture was heated at reflux for 18 hours, then cooled to room temperature, giving a precipitate that was filtered and washed with ethanol to yield the title compound (1.02 g, 40%) as a bronze solid. δ_{H} (250 MHz; D₂O): 7.90 (2H, br d, Ar*H*), 7.42 (4H, s, Ar*H*), 2.45 (8H, br q, NCH₂), 1.23 (12H, br t, *CH*₃).

### Synthesis 11

### Ethyl-thioninium nitrate (ETN)

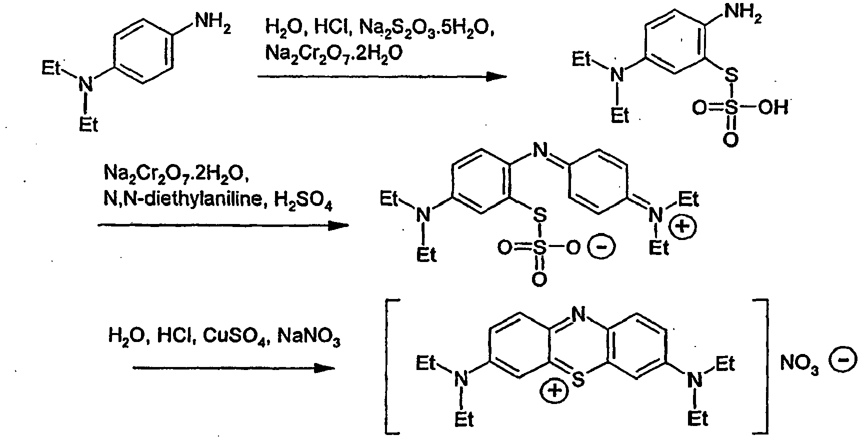

A stirred mixture of *N*,*N*-diethyl-*p*-phenylenediamine (10.0 g, 61 mmol) in aqueous hydrochloric acid (0.5 M, 200 cm³) was adjusted to pH 2 with aqueous sodium hydroxide (10%). The diamine solution was cooled to 5°C before the addition of aqueous Na₂S₂O₃.5H₂O (16.65 g, 67 mmol in 20 cm³ H₂O). An aqueous solution of Na₂Cr₂O₇.2H₂O (7.27 g, 24 mmol in 35 cm³ of H₂O) was added dropwise to the mixture over a 15 minute period giving a black suspension. The suspension was stirred at 5°C for 1 hour (pH = 8.07, T = 3.7°C). A solution of *N*,*N*-diethylaniline (8.25 g, 61 mmol), H₂SO₄ (6 g) and water (10 cm³) was cooled to 5°C before addition to the suspension. An aqueous solution of Na₂Cr₂O₇.2H₂O (19.09 g, 64 mmol in 50 cm³ of H₂O) was then added dropwise to the mixture over a 20 minute period giving a thick dark green suspension. The mixture was stirred at 5°C for 2 hours (pH = 6.75, T = 6°C) before filtering. The green purple solid obtained was washed with water (2 x 50 cm³). The solid was slurried in aqueous hydrochloric acid (300 cm³, pH 2) giving a suspension with a pH = 6.37 at 22°C. To the suspension was added CuSO₄ (1.52 g, 6.1 mmol) and the mixture heated to 90°C wherein a deep blue solution formed. After stirring at this temperature for 1 hour, the mixture was cooled to 25°C and filtered. The solid was washed with water (2 x 50 cm³), and the filtrate was adjusted from pH 6.33 to pH 2.00, T = 25°C with hydrochloric acid (5 M). The deep blue solution was heated to 80°C and had sodium nitrate (50 g) added and was allowed to cool to 25°C slowly while stirring gently. The product was filtered as green needles (6.80 g, 28%). δ_{H} (250 MHz, CDCl₃): 1.36 (12H, t, 7, CH₃), 3.72 (8H, q, 7, NCH₂), 7.23 (2H, d, 9.5, ArH), 7.39 (2H, s, ArH), 7.89 (2H, d, 9.5, ArH); δ_{H} (62.9 MHz, CDCl₃): 152.5, 138.8, 135.7, 135.6, 118.1, 106.4, 46.6, 12.9.

### Synthesis 12

### 1,9-Diethyl-methylthioninium chloride (DEMTC)

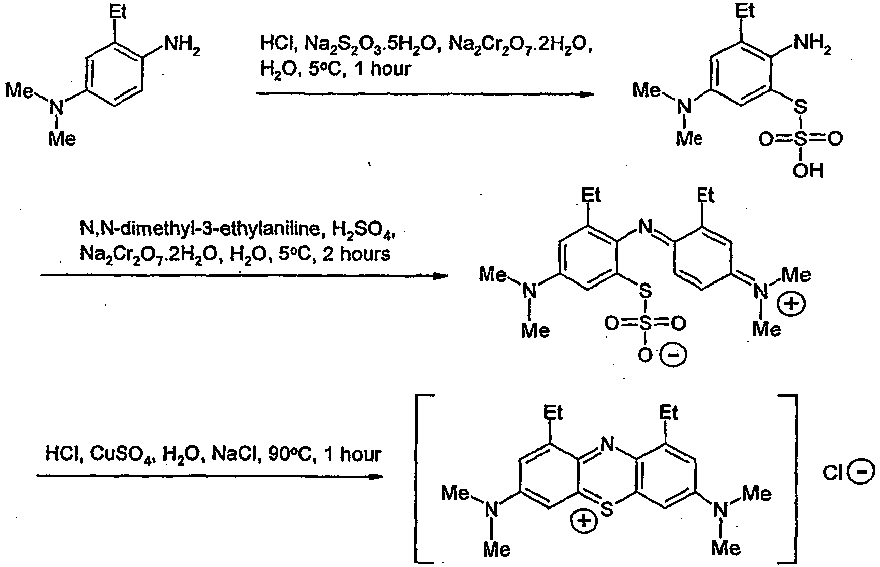

N,N-Dimethyl-3-ethyl-p-phenylenediamine (5.15 g, 31.4 mmol) was dissolved in aqueous hydrochloric acid (0.5 M, 100 cm³). The resulting solution was adjusted to pH 2 with aqueous sodium hydroxide (10%) and cooled to 5°C. An aqueous solution of Na₂S₂O₃.5H₂O (8.57 g, 34.6 mmol in 10 cm³ of water) was added drop-wise to the stirred solution. An aqueous solution of Na₂Cr₂O₇.2H₂O (3.74 g, 12.6 mmol in 18 cm³ of water) was added drop-wise over a 15 minute period giving a black suspension. Stirring was continued at 5°C for 1 hour. N,N-Dimethyl-3-ethylaniline (4.68 g, 31.4 mmol) was dissolved in aqueous sulphuric acid (3.00 g in 5 cm³ of water) and the resulting solution cooled to 5°C before addition to the reaction mixture. An aqueous solution of Na₂Cr₂O₇.2H₂O (9.83 g, 33.0 mmol in 25 cm³ of water) was added drop-wise over a 20 minute period giving a dark green suspension. Stirring was maintained at 5°C for a further 2 hours before the solid was collected by filtration. The solid was washed with water (3 x 30 cm³) before it was slurried in aqueous hydrochloric acid (150 cm³, pH 2). CuSO₄ (785 mg, 3.14 mmol) was added to the suspension and the mixture heated to 90°C for 1 hour. The resulting deep blue mixture was filtered and the solid washed with water (3 x 40 cm³). The filtrate was adjusted to pH 2 with hydrochloric acid (1 M). The solution was heated to 80°C before NaCl (2.00 g, 34.5 mmol) was added. The solution was allowed to cool to room temperature where a precipitate was deposited. The solid was collected by filtration to give the title compound as a dark green solid (1.89 g, 16%). δ_{H} (250 MHz; D₂O): 6.65 (2H, s, ArH), 6.23 (2H, s, ArH), 2.92 (12H, s, NCH₃), 2.56 (4H, q, 7.5, CH₂), 0.99 (6H, t, 7.5, CH₃); vₘₐₓ (KBr)/cm⁻¹ 3408 (CH), 2613 (CH), 1606 (C=N), 1399 (*CH*), 1316 (C*H*)*.* MS (ESI): 340.4 [100% (M-Cl)⁺].

This synthesis represents an alternative to Synthesis 3 above. The principal advantages of this synthesis compared to Synthesis 3 above are: (a) higher yield, (b) it can be performed at greater concentrations making it more suitable for use on an industrial scale, and (c) it does not produce H₂S, and so is cheaper and simpler when performed on an industrial scale.

### Synthesis 13

### Ethyl-thioninium nitrate (ETN)

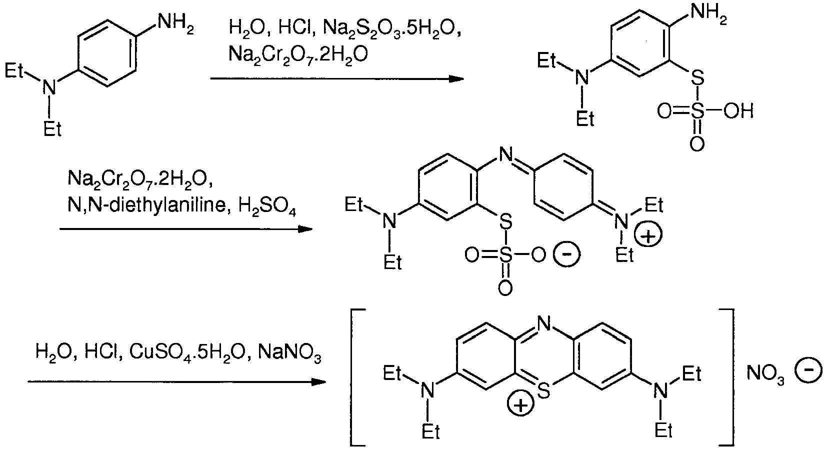

To a solution of *N*,*N*-diethyl-*p*-phenylene (6.61 g, 40 mmol) and aqueous hydrochloric acid (0.5 M, 132 mL) pre-cooled to 5°C was added aqueous sodium thiosulfate (10.91 g, 44 mmol in 15 mL of H₂O) in one portion. Sodium dichromate (4.96 g, 15.75 mmol in 10 mL of H₂O) was added drop-wise over a 10 minute period and the black solution was stirred for 1 hour at 5°C (pH = 8.22, Temp. = 1.8°C). Meanwhile a heterogeneous solution containing *N*,*N*-diethylaniline (5.96 g, 40 mmol), sulphuric acid (3.96 g), and water (6.60 mL) was prepared, cooled to 5°C, and then added to the stirring black suspension in one portion. A second sodium dichromate (12.52 g, 42 mmol in 40 mL of H₂O) solution was added drop-wise over a 15 minute period and the dark green suspension which formed was left stirring at 5°C for 2 hours (pH = 7.23, Temp. = 8.0°C). Sodium dithionite (1.48 g, 8.44 mmol) as an aqueous solution was added in one portion and the mixture was stirred for 15 minutes to warm to room temperature (22°C). The resulting reaction mixture was then used in one or the other of the following two alternatives.

*In one alternative (Filtration of BG (two-pot)):* The dark green solid which formed was filtered off, washed with water (2 x 50 mL) and sucked to reasonable dryness (30 minutes). The dark green solid was stirred in water acidified to pH 2 with hydrochloric acid and CuSO₄.5H₂O (0.99 g, 4.00 mmol) was added. The solution was heated to 90°C and stirred for 1 hour at this temperature resulting in the formation of an intense blue solution. Heating was stopped and the reaction mixture was left to cool to room temperature. The blue solution was filtered to remove insolubles and the solid washed with water (2 x 50 mL). The filtrate was acidified to pH 2 with hydrochloric acid (5 M, 5 mL) and NaNO₃ (33 g, 388 mmol) was added in one portion. The solution was heated to 80°C and left to cool to room temperature with gentle stirring. The green needles which resulted were filtered from the solution and sucked to dryness to give the title compound (4.38g; 28%). Mp 178°C; vₘₐₓ(KBr)/cm⁻¹ 3564 (CH), 3463 (CH), 3048 (CH), 2975 (CH), 1522 (C=C), 1489 (CH), 1370 (CH); δ_{H} (250 MHz, CDCl₃) 1.36 (12H, t, 7, CH₃), 3.72 (8H, q, 7 NCH₂), 7.23 (2H, d, 9.5, ArH), 7.39 (2H, s, ArH), 7.89 (2H, d 9.5, ArH); δ_{c} (62.9MHz, CDCl₃) 152.5, 138.8, 135.7, 135.6, 118.1, 106.4, 46.6, 12.9.

*In another alternative (No-filtration of BG (one-pot)):* Once the solution had warmed, CuSO₄.5H₂O (0.99 g, 4.00 mmol) was added. The solution was then heated to 90°C and stirred at this temperature for 1 hour, after which the solution had turned deep blue. The blue solution was allowed to cool to room temperature and filtered to remove insolubles. The filtrate was adjusted to pH 2 with hydrochloric acid (5 M, 20 mL approx.) and NaNO₃ (33 g, 388 mmol) was added. The stirring solution was heated to 80°C and left to cool overnight with gentle stirring. The resulting green needles were filtered from solution and sucked to dryness to give the title compound (1g, 5%). Mp 178°C; vₘₐₓ(KBr)/cm⁻¹ 3564 (CH), 3463 (CH), 3048 (CH), 2975 (CH), 1522 (C=C), 1489 (CH), 1370 (CH); δ_{H} (250 MHz, CDCl₃) 1.36 (12H, t, 7, CH₃), 3.72 (8H, q, 7 NCH₂), 7.23 (2H, d, 9.5, ArH), 7.39 (2H, s, ArH), 7.89 (2H, d 9.5, ArH); δ_{c} (62.9MHz, CDCl₃) 152.5, 138.8, 135.7, 135.6, 118.1, 106.4, 46.6, 12.9.

### Example 2 - Activity and Therapeutic Index

### In vitro assay for establishing B50

This is described in detail in WO 96/30766. Briefly, a fragment of tau corresponding to the core repeat domain, which has been adsorbed to a solid phase substrate, is able to capture soluble full-length tau and bind tau with high affinity. This association confers stability against proteolytic digestion of the aggregated tau molecules. The process is self-propagating, and can be blocked selectively by prototype pharmaceutical agents.

More specifically, truncated tau (residues 297-390; dGA) diluted in carbonate buffer (pH 9.6) was bound to the assay plate, and full-length tau (T40) was added in the aqueous phase. The aqueous phase binding buffer contained 0.05% Tween-20 and 1% gelatine in phosphate-buffered saline (pH7.4). Bound tau was detected using mAb 499 that recognises an N-terminal epitope within the aqueous phase full-length tau but that fails to recognise the solid phase-bound truncated tau fragment.

The concentration of compound required to inhibit the tau-tau binding by 50% is referred to as the B50 value.

### Cell-based assay for establishing EC50

The process is described in more detail in WO 02/055720. In essence, fibroblast cells (3T6) express full-length tau ("T40") under control of an inducible promotor, and low constitutive levels of the PHF-core tau fragment (12 kD fragment). When T40 expression is induced, it undergoes aggregation-dependent truncation within the cell, N-terminally at ∼ αα 295 and C-terminally at ∼ αα 390, thereby producing higher levels of the 12 kD PHF-core domain fragment. Production of the 12 kD fragment can be blocked in a dose-dependent manner by tau-aggregation inhibitors. Indeed the quantitation of inhibitory activity of compounds with respect to proteolytic generation of the 12 kD fragment within cells can be described entirely in terms of the same parameters which describe inhibition of tau-tau binding in vitro. That is, the extent of proteolytic generation of the 12 kD fragment within cells is determined entirely by the extent to tau-tau binding through the repeat domain. The availability of the relevant proteases within the cell is non-limiting.

Results are expressed as the concentration at which there is a 50% inhibition of generation of the 12 kD fragment. This is referred to as the EC50 value.

### Toxicity in cells - LD50 and therapeutic index (Rxl)

Toxicity of the compounds described herein was assessed in the cell based assay used to assess EC50. Toxicity was measured by cell numbers after 24 hrs exposure to the compound using a lactate dehydrogenase assay kit TOX-7(Sigma Biosciences) according to the manufacturer's instructions after lysis of remaining cells. Alternatively a kit from Promega UK (CytoTox 96) was used, again according to the manufacturer's instructions.

The therapeutic index (Rxl) was calculated as follows: Rxl = LD50 / EC50.

| | Compound | B50 (µM) | EC50 (µM) | LD50 (µM) | Rxl |
|---|---|---|---|---|---|
| | MTC | 218 ± 20.1 (6) | 0.59 ± 0.04 (69) | 65.0 ± 5.0 (38) | 110 |
| | DMMTC | 3.4 ± 0.2 (2) | 0.04 ± 0.004 (22) | 2.7 ± 1.2 (6) | 67 |
| A | ETC | 49.0 ± 8.5 (10) | 0.07 ± 0.007 (53) | 32.0 ± 4.0 (26) | 480 |
| B | DEMTC | 26.2 ± 5.3 (6) | 0.0016 ± 0.0006 (13) | 3.3 ± 0.6 (22) | 2,173 |
| C | DMETC | 4.5 ± 0.3 (3) | 0.004 ± 0.001 (6) | 4.2 ± 2.2 (4) | 1,048 |
| D | DEETC | 3.7 ± 0.5 (3) | 0.0006 ± 0.0003 (3) | 1.6 ± 0.4 (13) | 2,667 |
| F | ETZ | 145.4 ± 5.7 (5) | 0.06 ± 0.01 (6) | 39.0 ± 7.0 (4) | 670 |
| G | MTI | 382 | 0.72 | 120 | 168 |
| H | MTI.HI | 271 | 0.70 | 120 | 168 |
| I | ETI | >500 | 0.06 | - | - |
| J | ETI.HI | 83 | - | - | - |
| L | ETN | > 500 (2) | 0.04 (1) | 13.0 ± 0.5. (2) | 325 |

## Claims

1. A compound for use in a method of treatment or prophylaxis of the human or animal body by therapy, wherein the compound is:
(C) 1,9-dimethyl-ethyl-thioninium chloride (DMETC),
(D) 1,9-diethyl-ethyl-thioninium chloride (DEETC),
(I) ethyl-thioninium iodide (ETI),
(J) ethyl-thioninium iodide hydrogen iodide mixed salt (ETI.HI), or
(L) ethyl-thioninium nitrate (ETN).

2. A compound for use in a method of treatment or prophylaxis of a tauopathy condition in a patient, or a disease of tau protein aggregation in a patient, wherein the compound is:
(C) 1,9-dimethyl-ethyl-thioninium chloride (DMETC),
(D) 1,9-diethyl-ethyl-thioninium chloride (DEETC),
(I) ethyl-thioninium iodide (ETI),
(J) ethyl-thioninium iodide hydrogen iodide mixed salt (ETI.HI), or
(L) ethyl-thioninium nitrate (ETN).

3. A compound for use in a method of treatment or prophylaxis of Alzheimer's disease (AD), Pick's disease, Progressive Supranuclear Palsy (PSP), fronto-temporal dementia (FTD), fronto-temporal dementia with parkinsonism linked to chromosome 17 (FTDP-17), disinhibition-dementia-parkinsonism-amyotrophy complex (DDPAC), pallido-ponto-nigral degeneration (PPND), Guam-ALS syndrome, pallido-nigro-luysian degeneration (PNLD), or cortico-basal degeneration (CBD) in a patient, wherein the compound is:
(C) 1,9-dimethyl-ethyl-thioninium chloride (DMETC),
(D) 1,9-diethyl-ethyl-thioninium chloride (DEETC),
(I) ethyl-thioninium iodide (ETI),
(J) ethyl-thioninium iodide hydrogen iodide mixed salt (ETI.HI), or
(L) ethyl-thioninium nitrate (ETN).

4. A compound according to claim 3, for use in a method of treatment or prophylaxis of Alzheimer's disease (AD) in a patient, wherein the compound is:
(C) 1,9-dimethyl-ethyl-thioninium chloride (DMETC),
(D) 1,9-diethyl-ethyl-thioninium chloride (DEETC),
(I) ethyl-thioninium iodide (ETI),
(J) ethyl-thioninium iodide hydrogen iodide mixed salt (ETI.HI), or
(L) ethyl-thioninium nitrate (ETN).

5. A compound according to claim 4, wherein the compound is:
(C) 1,9-dimethyl-ethyl-thioninium chloride (DMETC).

6. A compound according to claim 4, wherein the compound is:
(D) 1,9-diethyl-ethyl-thioninium chloride (DEETC).

7. A compound according to claim 4, wherein the compound is:
(I) ethyl-thioninium iodide (ETI).

8. A compound according to claim 4, wherein the compound is:
(J) ethyl-thioninium iodide hydrogen iodide mixed salt (ETI.HI).

9. A compound according to claim 4, wherein the compound is:
(L) ethyl-thioninium nitrate (ETN).

10. A compound according to any one of claims 1 to 9, wherein the treatment or prophylaxis comprises administration of the compound according to either one of the following dosage regimes: about 50 or about 75 mg, 3 or 4 times daily; or, about 100 or about 125 mg, 2 times daily.

11. A compound for use in a method of treatment or prophylaxis of a tauopathy condition in a patient, or a disease of tau protein aggregation in a patient, wherein the compound is:
(A) ethyl-thioninium chloride (ETC),
(B) 1,9-diethyl-methyl-thioninium chloride (DEMTC), or
(F) ethyl-thioninium chloride zinc chloride mixed salt (ETZ);
wherein the treatment or prophylaxis comprises administration of the compound according to the following dosage regime: about 50 or about 75 mg, 3 times daily; about 50 mg, 4 times daily; or about 100 or about 125 mg, 2 times daily.

12. A compound for use in a method of treatment or prophylaxis of Alzheimer's disease (AD), Pick's disease, Progressive Supranuclear Palsy (PSP), fronto-temporal dementia (FTD), fronto-temporal dementia with parkinsonism linked to chromosome 17 (FTDP-17), disinhibition-dementia-parkinsonism-amyotrophy complex (DDPAC), pallido-ponto-nigral degeneration (PPND), Guam-ALS syndrome, pallido-nigro-luysian degeneration (PNLD), or cortico-basal degeneration (CBD) in a patient, wherein the compound is:
(A) ethyl-thioninium chloride (ETC),
(B) 1,9-diethyl-methyl-thioninium chloride (DEMTC), or
(F) ethyl-thioninium chloride zinc chloride mixed salt (ETZ);
wherein the treatment or prophylaxis comprises administration of the compound according to the following dosage regime: about 50 or about 75 mg, 3 times daily; about 50 mg, 4 times daily; or about 100 or about 125 mg, 2 times daily.

13. A compound according to claim 12, for use in a method of treatment or prophylaxis of Alzheimer's disease (AD) in a patient, wherein the compound is:
(A) ethyl-thioninium chloride (ETC),
(B) 1,9-diethyl-methyl-thioninium chloride (DEMTC), or
(F) ethyl-thioninium chloride zinc chloride mixed salt (ETZ);
wherein the treatment or prophylaxis comprises administration of the compound according to the following dosage regime: about 50 or about 75 mg, 3 times daily; about 50 mg, 4 times daily; or about 100 or about 125 mg, 2 times daily.

14. A compound according to claim 13, wherein the compound is:
(A) ethyl-thioninium chloride (ETC).

15. A compound according to claim 12, wherein the compound is:
(B) 1,9-diethyl-methyl-thioninium chloride (DEMTC).

16. A compound according to claim 12, wherein the compound is:
(F) ethyl-thioninium chloride zinc chloride mixed salt (ETZ).

17. A compound according to any one of claims 1 to 16, wherein the compound is provided in the form of a dosage unit comprising the compound in an amount of from 20 to 300 mg and a pharmaceutically acceptable carrier, diluent, or excipient.

18. A compound according to any one of claims 1 to 17, wherein the treatment or prophylaxis comprises oral administration of the compound.

19. A compound according to any one of claims 1 to 18, wherein the treatment or prophylaxis further comprises treatment with a cholinesterase inhibitor, Donepezil , Rivastigmine, Galantamine, an NMDA receptor antagonist, Memantine, a muscarinic receptor agonist, or an inhibitor of amyloid precursor protein processing to beta-amyloid.

20. Use of a compound in the manufacture of a medicament for use in a method of treatment or prophylaxis of a tauopathy condition in a patient, or a disease of tau protein aggregation in a patient, wherein the compound is:
(C) 1,9-dimethyl-ethyl-thioninium chloride (DMETC),
(D) 1,9-diethyl-ethyl-thioninium chloride (DEETC),
(I) ethyl-thioninium iodide (ETI),
(J) ethyl-thioninium iodide hydrogen iodide mixed salt (ETI.HI), or
(L) ethyl-thioninium nitrate (ETN).

21. Use of a compound in the manufacture of a medicament for use in a method of treatment or prophylaxis of Alzheimer's disease (AD), Pick's disease, Progressive Supranuclear Palsy (PSP), fronto-temporal dementia (FTD), fronto-temporal dementia with parkinsonism linked to chromosome 17 (FTDP-17), disinhibition-dementia-parkinsonism-amyotrophy complex (DDPAC), pallido-ponto-nigral degeneration (PPND), Guam-ALS syndrome, pallido-nigro-luysian degeneration (PNLD), or cortico-basal degeneration (CBD) in a patient, wherein the compound is:
(C) 1,9-dimethyl-ethyl-thioninium chloride (DMETC),
(D) 1,9-diethyl-ethyl-thioninium chloride (DEETC),
(I) ethyl-thioninium iodide (ETI),
(J) ethyl-thioninium iodide hydrogen iodide mixed salt (ETI.HI), or
(L) ethyl-thioninium nitrate (ETN).

22. Use of a compound according to claim 21 in the manufacture of a medicament for use in a method of treatment or prophylaxis of Alzheimer's disease (AD) in a patient, wherein the compound is:
(C) 1,9-dimethyl-ethyl-thioninium chloride (DMETC),
(D) 1,9-diethyl-ethyl-thioninium chloride (DEETC),
(I) ethyl-thioninium iodide (ETI),
(J) ethyl-thioninium iodide hydrogen iodide mixed salt (ETI.HI), or
(L) ethyl-thioninium nitrate (ETN).

23. Use of a compound according to claim 22, wherein the compound is:
(C) 1,9-dimethyl-ethyl-thioninium chloride (DMETC).

24. Use of a compound according to claim 22, wherein the compound is:
(D) 1,9-diethyl-ethyl-thioninium chloride (DEETC).

25. Use of a compound according to claim 22, wherein the compound is:
(I) ethyl-thioninium iodide (ETI).

26. Use of a compound according to claim 22, wherein the compound is:
(J) ethyl-thioninium iodide hydrogen iodide mixed salt (ETI.HI).

27. Use of a compound according to claim 22, wherein the compound is:
(L) ethyl-thioninium nitrate (ETN).

28. Use according to any one of claims 21 to 28, wherein the treatment or prophylaxis comprises administration of the compound according to the following dosage regime: about 50 or about 75 mg, 3 or 4 times daily; or, about 100 or about 125 mg, 2 times daily.

29. Use of a compound in the manufacture of a medicament for use in a method of treatment or prophylaxis of a tauopathy condition in a patient, or a disease of tau protein aggregation in a patient, wherein the compound is:
(A) ethyl-thioninium chloride (ETC),
(B) 1,9-diethyl-methyl-thioninium chloride (DEMTC), or
(F) ethyl-thioninium chloride zinc chloride mixed salt (ETZ);
wherein the treatment or prophylaxis comprises administration of the compound according to the following dosage regime: about 50 or about 75 mg, 3 times daily; about 50 mg, 4 times daily; or about 100 or about 125 mg, 2 times daily.

30. Use of a compound in the manufacture of a medicament for use in a method of treatment or prophylaxis of Alzheimer's disease (AD), Pick's disease, Progressive Supranuclear Palsy (PSP), fronto-temporal dementia (FTD), fronto-temporal dementia with parkinsonism linked to chromosome 17 (FTDP-17), disinhibition-dementia-parkinsonism-amyotrophy complex (DDPAC), pallido-ponto-nigral degeneration (PPND), Guam-ALS syndrome, pallido-nigro-luysian degeneration (PNLD), or cortico-basal degeneration (CBD) in a patient, wherein the compound is:
(A) ethyl-thioninium chloride (ETC),
(B) 1,9-diethyl-methyl-thioninium chloride (DEMTC), or
(F) ethyl-thioninium chloride zinc chloride mixed salt (ETZ);
wherein the treatment or prophylaxis comprises administration of the compound according to the following dosage regime: about 50 or about 75 mg, 3 times daily; about 50 mg, 4 times daily; or about 100 or about 125 mg, 2 times daily.

31. Use of a compound according to claim 30 in the manufacture of a medicament for use in a method of treatment or prophylaxis of Alzheimer's disease (AD) in a patient, wherein the compound is:
(A) ethyl-thioninium chloride (ETC),
(B) 1,9-diethyl-methyl-thioninium chloride (DEMTC), or
(F) ethyl-thioninium chloride zinc chloride mixed salt (ETZ);
wherein the treatment or prophylaxis comprises administration of the compound according to the following dosage regime: about 50 or about 75 mg, 3 times daily; about 50 mg, 4 times daily; or about 100 or about 125 mg, 2 times daily.

32. Use of a compound according to claim 31, wherein the compound is:
(A) ethyl-thioninium chloride (ETC).

33. Use of a compound according to claim 31, wherein the compound is:
(B) 1,9-diethyl-methyl-thioninium chloride (DEMTC).

34. Use of a compound according to claim 31, wherein the compound is:
(F) ethyl-thioninium chloride zinc chloride mixed salt (ETZ).

35. Use according to any one of claims 20 to 34, wherein the medicament is a dosage unit comprising the compound in an amount of from 20 to 300 mg and a pharmaceutically acceptable carrier, diluent, or excipient.

36. Use according to any one of claims 20 to 35, wherein the treatment or prophylaxis comprises oral administration of the compound.

37. Use according to any one of claims 20 to 36, wherein the treatment or prophylaxis further comprises treatment with a cholinesterase inhibitor, Donepezil, Rivastigmine, or Galantamine, an NMDA receptor antagonist, Memantine, a muscarinic receptor agonist, or an inhibitor of amyloid precursor protein processing to beta-amyloid.

## Patentansprüche

1. Verbindung zur Verwendung in einem Verfahren zur Behandlung oder Prophylaxe des menschlichen oder tierischen Körpers durch Therapie, wobei die Verbindung:
(C) 1,9-Dimethylethylthioniniumchlorid (DMETC),
(D) 1,9-Diethylethylthioniniumchlorid (DEETC),
(I) Ethylthioniniumiodid (ETI),
(J) Ethylthioniniumiodid-Hydrogeniodid-Mischsalz (ETI.HI), oder
(L) Ethylthioniniumnitrat (ETN) ist.

2. Verbindung zur Verwendung in einem Verfahren zur Behandlung oder Prophylaxe eines tauopatischen Leidens bei einem Patienten oder einer Erkrankung durch Tau-Protein-Ansammlung bei einem Patienten, wobei die Verbindung:
(C) 1,9-Dimethylethylthioniniumchlorid (DMETC),
(D) 1,9-Diethylethylthioniniumchlorid (DEETC),
(I) Ethylthioniniumiodid (ETI),
(J) Ethylthioniniumiodid-Hydrogeniodid-Mischsalz (ETI.HI), oder
(L) Ethylthioniniumnitrat (ETN) ist.

3. Verbindung zur Verwendung in einem Verfahren zur Behandlung oder Prophylaxe der Alzheimer-Krankheit (AK), der Pick-Krankheit, der progressiven supernukleären Blickparese (PSP), der frontotemporalen Demenz und Parkinsonismus des Chromosoms 17 (FTDP-17), des Enthemmung-Demenz-Parkinsonismus-Amyotrophie-Komplexes (DDPAC), der pallido-ponto-nigralen Degeneration (PPND), des Guam-ALS-Syndroms, der Pallido-Nigro-Luysia-Degeneration (PNLD) oder der cortico-basalen Degeneration (CBD) bei einem Patienten, wobei die Verbindung:
(C) 1,9-Dimethylethylthioniniumchlorid (DMETC),
(D) 1,9-Diethylethylthioniniumchlorid (DEETC),
(I) Ethylthioniniumiodid (ETI),
(J) Ethylthioniniumiodid-Hydrogeniodid-Mischsalz (ETI.HI), oder
(L) Ethylthioniniumnitrat (ETN) ist.

4. Verbindung nach Anspruch 3 zur Verwendung in einem Verfahren zur Behandlung oder Prophylaxe der Alzheimer-Krankheit (AK) bei einem Patienten, wobei die Verbindung:
(C) 1,9-Dimethylethylthioniniumchlorid (DMETC),
(D) 1,9-Diethylethylthioniniumchlorid (DEETC),
(I) Ethylthioniniumiodid (ETI),
(J) Ethylthioniniumiodid-Hydrogeniodid-Mischsalz (ETI.HI), oder
(L) Ethylthioniniumnitrat (ETN) ist.

5. Verbindung nach Anspruch 4, wobei die Verbindung:
(C) 1,9-Dimethylethylthioniniumchlorid (DMETC) ist.

6. Verbindung nach Anspruch 4, wobei die Verbindung:
(D) 1,9-Diethylethylthioniniumchlorid (DEETC) ist.

7. Verbindung nach Anspruch 4, wobei die Verbindung:
(I) Ethylthioniniumiodid (ETI) ist.

8. Verbindung nach Anspruch 4, wobei die Verbindung:
(J) Ethylthioniniumiodid-Hydrogeniodid-Mischsalz (ETI.HI) ist.

9. Verbindung nach Anspruch 4, wobei die Verbindung:
(L) Ethylthioniniumnitrat (ETN) ist.

10. Verbindung nach einem der Ansprüche 1 bis 9, wobei die Behandlung oder Phrophylaxe die Verabreichung der Verbindung nach einem der folgenden Dosierungsschemata umfasst: etwa 50 oder etwa 75 mg drei- oder viermal täglich; oder etwa 100 oder etwa 125 mg zweimal täglich.

11. Verbindung zur Verwendung in einem Verfahren zur Behandlung oder Prophylaxe eines tauopatischen Leidens bei einem Patienten oder einer Erkrankung durch Tau-Protein-Ansammlung bei einem Patienten, wobei die Verbindung:
(A) Ethylthioniniumchlorid (ETC),
(B) 1,9-Diethylmethylthioniniumchlorid (DEMTC), oder
(F) Ethylthioniniumchloride-Zinkchlorid-Mischsalz (ETZ) ist;
wobei die Behandlung oder Prophylaxe die Verabreichung der Verbindung nach einem der folgenden Dosierungsschemata umfasst: etwa 50 oder etwa 75 mg dreimal täglich; etwa 50 mg viermal täglich; oder etwa 100 oder etwa 125 mg zweimal täglich.

12. Verbindung zur Verwendung in einem Verfahren zur Behandlung oder Prophylaxe der Alzheimer-Krankheit (AK), der Pick-Krankheit, der progressiven supernukleären Blickparese (PSP), der frontotemporalen Demenz und Parkinsonismus des Chromosoms 17 (FTDP-17), des Enthemmung-Demenz-Parkinsonismus-Amyotrophie-Komplexes (DDPAC), der pallido-ponto-nigralen Degeneration (PPND), des Guam-ALS-Syndroms, der Pallido-Nigro-Luysia-Degeneration (PNLD) oder der cortico-basalen Degeneration (CBD) bei einem Patienten, wobei die Verbindung:
(A) Ethylthioniniumchlorid (ETC),
(B) 1,9-Diethylmethylthioniniumchlorid (DEMTC), oder
(F) Ethylthioniniumchloride-Zinkchlorid-Mischsalz (ETZ) ist;
wobei die Behandlung oder Prophylaxe die Verabreichung der Verbindung nach einem der folgenden Dosierungsschemata umfasst: etwa 50 oder etwa 75 mg dreimal täglich; etwa 50 mg viermal täglich; oder etwa 100 oder etwa 125 mg zweimal täglich.

13. Verbindung nach Anspruch 12 Verfahren zur Behandlung oder Prophylaxe der Alzheimer-Krankheit (AK) bei einem Patienten, wobei die Verbindung:
(A) Ethylthioniniumchlorid (ETC),
(B) 1,9-Diethylmethylthioniniumchlorid (DEMTC), oder
(F) Ethylthioniniumchloride-Zinkchlorid-Mischsalz (ETZ) ist;
wobei die Behandlung oder Prophylaxe die Verabreichung der Verbindung nach einem der folgenden Dosierungsschemata umfasst: etwa 50 oder etwa 75 mg dreimal täglich; etwa 50 mg viermal täglich; oder etwa 100 oder etwa 125 mg zweimal täglich.

14. Verbindung nach Anspruch 13, wobei die Verbindung:
(A) Ethylthioniniumchlorid (ETC) ist.

15. Verbindung nach Anspruch 12, wobei die Verbindung:
(B) 1,9-Diethylmethylthioniniumchlorid (DEMTC) ist.

16. Verbindung nach Anspruch 12, wobei die Verbindung:
(F) Ethylthioniniumchlorid-Zinkchlorid-Mischsalz (ETZ) ist.

17. Verbindung nach einem der Ansprüche 1 bis 16, wobei die Verbindung in Form einer Dosierungseinheit bereitgestellt wird, die die Verbindung in einer Menge von 20 bis 300 mg sowie einen pharmazeutisch annehmbaren Träger, Verdünner oder Exzipienten umfasst.

18. Verbindung nach einem der Ansprüche 1 bis 17, wobei die Behandlung oder Prophylaxe die orale Verabreichung der Verbindung umfasst.

19. Verbindung nach einem der Ansprüche 1 bis 18, wobei die Behandlung oder Prophylaxe zudem die Behandlung mit einem Cholinesterasehemmer, Donepezil, Rivastigmin, Galantamin einen NMDF-Rezeptorantagonisten, Memantin, einem muskarinischen Rezeptoragonisten oder einem Inhibitor der Verarbeitung von amyoloidem Vorläuferprotein zu β-Amyloid umfasst.

20. Verwendung einer Verbindung zur Herstellung eines Medikaments zur Verwendung in einem Verfahren zur Behandlung oder Prophylaxe eines tauopatischen Leidens bei einem Patienten oder einer Erkrankung durch Tau-Protein-Ansammlung bei einem Patienten, wobei die Verbindung:
(C) 1,9-Dimethylethylthioniniumchlorid (DMETC),
(D) 1,9-Diethylethylthioniniumchlorid (DEETC),
(I) Ethylthioniniumiodid (ETI),
(J) Ethylthioniniumiodid-Hydrogeniodid-Mischsalz (ETI.HI), oder
(L) Ethylthioniniumnitrat (ETN) ist.

21. Verwendung einer Verbindung zur Herstellung eines Medikaments zur Verwendung in einem Verfahren zur Behandlung oder Prophylaxe der Alzheimer-Krankheit (AK), der Pick-Krankheit, der progressiven supernukleären Blickparese (PSP), der frontotemporalen Demenz und Parkinsonismus des Chromosoms 17(FTDP-17), des Enthemmung-Demenz-Parkinsonismus-Amyotrophie-Komplexes (DDPAC), der pallido-ponto-nigralen Degeneration (PPND), des Guam-ALS-Syndroms, der Pallido-Nigro-Luysia-Degeneration (PNLD) oder der cortico-basalen Degeneration (CBD) bei einem Patienten, wobei die Verbindung:
(C) 1,9-Dimethylethylthioniniumchlorid (DMETC),
(D) 1,9-Diethylethylthioniniumchlorid (DEETC),
(I) Ethylthioniniumiodid (ETI),
(J) Ethylthioniniumiodid-Hydrogeniodid-Mischsalz (ETI.HI), oder
(L) Ethylthioniniumnitrat (ETN) ist.

22. Verwendung einer Verbindung nach Anspruch 21 zur Herstellung eines Medikaments zur Verwendung in einem Verfahren zur Behandlung oder Prophylaxe der Alzheimer-Krankheit (AK) bei einem Patienten, wobei die Verbindung:
(C) 1,9-Dimethylethylthioniniumchlorid (DMETC),
(D) 1,9-Diethylethylthioniniumchlorid (DEETC),
(I) Ethylthioniniumiodid (ETI),
(J) Ethylthioniniumiodid-Hydrogeniodid-Mischsalz (ETI.HI), oder
(L) Ethylthioniniumnitrat (ETN) ist.

23. Verwendung einer Verbindung nach Anspruch 22, wobei die Verbindung (C) 1,9-Dimethylethylthioniniumchlorid (DMETC) ist.

24. Verwendung einer Verbindung nach Anspruch 22, wobei die Verbindung (D) 1,9-Diethylethylthioniniumchlorid (DEETC) ist.

25. Verwendung einer Verbindung nach Anspruch 22, wobei die Verbindung (I) Ethylthioniniumiodid (ETI) ist.

26. Verwendung einer Verbindung nach Anspruch 22, wobei die Verbindung (J) Ethylthioniniumiodid-Hydrogeniodid-Mischsalz (ETI.HI) ist.

27. Verwendung einer Verbindung nach Anspruch 22, wobei die Verbindung (L) Ethylthioniniumnitrat (ETN) ist.

28. Verwendung nach einem der Ansprüche 21 bis 28, wobei die Behandlung oder Prophylaxe die Verabreichung der Verbindung nach dem folgenden Dosierungsschema umfasst: etwa 50 oder etwa 75 mg drei- bis viermal täglich; oder etwa 100 oder etwa 125 mg zweimal täglich.

29. Verwendung einer Verbindung zur Herstellung eines Medikaments zur Verwendung in einem Verfahren zur Behandlung oder Prophylaxe eines tauopatischen Leidens bei einem Patienten oder einer Erkrankung durch Tau-Protein-Ansammlung bei einem Patienten, wobei die Verbindung:
(A) Ethylthioniniumchlorid (ETC),
(B) 1,9-Diethylmethylthioniniumchlorid (DEMTC), oder
(F) Ethylthioniniumchloride-Zinkchlorid-Mischsalz (ETZ) ist;
wobei die Behandlung oder Prophylaxe die Verabreichung der Verbindung nach dem folgenden Dosierungsschema umfasst: etwa 50 oder etwa 75 mg dreimal täglich; etwa 50 mg viermal täglich; oder etwa 100 oder etwa 125 mg zweimal täglich.

30. Verwendung einer Verbindung zur Herstellung eines Medikaments zur Verwendung in einem Verfahren zur Behandlung oder Prophylaxe der Alzheimer-Krankheit (AK), der Pick-Krankheit, der progressiven supernukleären Blickparese (PSP), der frontotemporalen Demenz und Parkinsonismus des Chromosoms 17(FTDP-17), des Enthemmung-Demenz-Parkinsonismus-Amyotrophie-Komplexes (DDPAC), der pallido-ponto-nigralen Degeneration (PPND), des Guam-ALS-Syndroms, der Pallido-Nigro-Luysia-Degeneration (PNLD) oder der cortico-basalen Degeneration (CBD) bei einem Patienten, wobei die Verbindung:
(A) Ethylthioniniumchlorid (ETC),
(B) 1,9-Diethylmethylthioniniumchlorid (DEMTC), oder
(F) Ethylthioniniumchloride-Zinkchlorid-Mischsalz (ETZ) ist;
wobei die Behandlung oder Prophylaxe die Verabreichung der Verbindung nach dem folgenden Dosierungsschema umfasst: etwa 50 oder etwa 75 mg dreimal täglich; etwa 50 mg viermal täglich; oder etwa 100 oder etwa 125 mg zweimal täglich.

31. Verwendung einer Verbindung nach Anspruch 30 zur Herstellung eines Medikaments zur Verwendung in einem Verfahren zur Behandlung oder Prophylaxe der Alzheimer-Krankheit (AK) bei einem Patienten, wobei die Verbindung:
(A) Ethylthioniniumchlorid (ETC),
(B) 1,9-Diethylmethylthioniniumchlorid (DEMTC), oder
(F) Ethylthioniniumchloride-Zinkchlorid-Mischsalz (ETZ) ist;
wobei die Behandlung oder Prophylaxe die Verabreichung der Verbindung nach dem folgenden Dosierungsschema umfasst: etwa 50 oder etwa 75 mg dreimal täglich; etwa 50 mg viermal täglich; oder etwa 100 oder etwa 125 mg zweimal täglich.

32. Verwendung einer Verbindung nach Anspruch 31, wobei die Verbindung:
(A) Ethylthioniniumchlorid (ETC) ist.

33. Verwendung einer Verbindung nach Anspruch 31, wobei die Verbindung:
(B) 1,9-Diethylmethylthioniniumchlorid (DEMTC) ist.

34. Verwendung einer Verbindung nach Anspruch 31, wobei die Verbindung:
(F) Ethylthioniniumchlorid-Zinkchlorid-Mischsalz (ETZ) ist.

35. Verwendung nach einem der Ansprüche 20 bis 24, wobei das Medikament eine Dosierungseinheit ist, die die Verbindung in einer Menge von 20 bis 300 mg sowie einen pharmazeutisch annehmbaren Träger, Verdünner oder Exzipienten umfasst.

36. Verwendung nach einem der Ansprüche 20 bis 35, wobei das Verfahren oder die Prophylaxe die orale Verabreichung der Verbindung umfasst.

37. Verwendung nach einem der Ansprüche 20 bis 36, wobei die Behandlung oder Prophylaxe zudem die Behandlung mit einem Cholinesterasehemmer, Donepezil, Rivastigmin, Galantamin einen NMDF-Rezeptorantagonisten, Memantin, einem muskarinischen Rezeptoragonisten oder einem Inhibitor der Verarbeitung von amyloiden Vorläuferprotein zu β-Amyloid umfasst.

## Revendications

1. Composé pour utilisation dans une méthode de traitement ou de prophylaxie du corps humain ou animal par thérapie, lequel composé est :
(C) le chlorure de 1,9-diméthyléthyl-thioninium (DMETC),
(D) le chlorure de 1,9-diéthyléthyl-thioninium (DEETC),
(I) l'iodure d'éthyl-thioninium (ETI),
(J) le sel mixte d'iodure d'éthyl-thioninium et d'iodure d'hydrogène (ETI.HI), ou
(L) le nitrate d'éthyl-thioninium (ETN).

2. Composé pour utilisation dans une méthode de traitement ou de prophylaxie d'un état de tauopathie chez un patient, ou d'une maladie d'agrégation de la protéine tau chez un patient, lequel composé est :
(C) le chlorure de 1,9-diméthyléthyl-thioninium (DMETC),
(D) le chlorure de 1,9-diéthyléthyl-thioninium (DEETC),
(I) l'iodure d'éthyl-thioninium (ETI),
(J) le sel mixte d'iodure d'éthyl-thioninium et d'iodure d'hydrogène (ETI.HI), ou
(L) le nitrate d'éthyl-thioninium (ETN).

3. Composé pour utilisation dans une méthode de traitement ou de prophylaxie de la maladie d'Alzheimer (AD), de la maladie de Pick, de la paralysie supranucléaire progressive (PSP), de la démence fronto-temporale (FTD), de la démence fronto-temporale avec un parkinsonisme liée au chromosome 17 (FTDP-17), du complexe de désinhibition-démence-parkinsonisme-amyotrophie (DDPAC), de la dégénérescence pallido-ponto-nigrale (PPND), du syndrome de Guam-ALS, de la dégénérescence pallido-nigro-luysienne (PNLD), ou de la dégénérescence cortico-basale (CBD) chez un patient, lequel composé est :
(C) le chlorure de 1,9-diméthyléthyl-thioninium (DMETC),
(D) le chlorure de 1,9-diéthyléthyl-thioninium (DEETC),
(I) l'iodure d'éthyl-thioninium (ETI),
(J) le sel mixte d'iodure d'éthyl-thioninium et d'iodure d'hydrogène (ETI.HI), ou
(L) le nitrate d'éthyl-thioninium (ETN).

4. Composé selon la revendication 3, pour utilisation dans une méthode de traitement ou de prophylaxie de la maladie d'Alzheimer (AD) chez un patient, lequel composé est :
(C) le chlorure de 1,9-diméthyléthyl-thioninium (DMETC),
(D) le chlorure de 1,9-diéthyléthyl-thioninium (DEETC),
(I) l'iodure d'éthyl-thioninium (ETI),
(J) le sel mixte d'iodure d'éthyl-thioninium et d'iodure d'hydrogène (ETI.HI), ou
(L) le nitrate d'éthyl-thioninium (ETN).

5. Composé selon la revendication 4, lequel composé est :
(C) le chlorure de 1,9-diméthyléthyl-thioninium (DMETC).

6. Composé selon la revendication 4, lequel composé est :
(D) le chlorure de 1,9-diéthyléthyl-thioninium (DEETC).

7. Composé selon la revendication 4, lequel composé est :
(I) l'iodure d'éthyl-thioninium (ETI).

8. Composé selon la revendication 4, lequel composé est :
(J) le sel mixte d'iodure d'éthyl-thioninium et d'iodure d'hydrogène (ETI.HI).

9. Composé selon la revendication 4, lequel composé est :
(L) le nitrate d'éthyl-thioninium (ETN).

10. Composé selon l'une quelconque des revendications 1 à 9, dans lequel le traitement ou la prophylaxie comprend l'administration du composé selon l'un ou l'autre des régimes posologiques suivants : environ 50 ou environ 75 mg, 3 ou 4 fois par jour ; et environ 100 ou environ 125 mg, 2 fois par jour.

11. Composé pour utilisation dans une méthode de traitement ou de prophylaxie d'un état de tauopathie chez un patient, ou d'une maladie d'agrégation de la protéine tau chez un patient, lequel composé est :
(A) le chlorure d'éthyl-thioninium (ETC),
(B) le chlorure de 1,9-diéthylméthyl-thioninium (DEMTC), ou
(F) le sel mixte de chlorure d'éthyl-thioninium et de chlorure de zinc (ETZ) ;
dans lequel le traitement ou la prophylaxie comprend l'administration du composé selon le régime posologique suivant : environ 50 ou environ 75 mg, 3 fois par jour ; environ 50 mg, 4 fois par jour ; ou environ 100 ou environ 125 mg, 2 fois par jour.

12. Composé pour utilisation dans une méthode de traitement ou de prophylaxie de la maladie d'Alzheimer (AD), de la maladie de Pick, de la paralysie supranucléaire progressive (PSP), de la démence fronto-temporale (FTD), de la démence fronto-temporale avec un parkinsonisme liée au chromosome 17 (FTDP-17), du complexe de désinhibition-démence-parkinsonisme-amyotrophie (DDPAC), de la dégénérescence pallido-ponto-nigrale (PPND), du syndrome de Guam-ALS, de la dégénérescence pallido-nigro-luysienne (PNLD), ou de la dégénérescence cortico-basale (CBD) chez un patient, lequel composé est :
(A) le chlorure d'éthyl-thioninium (ETC),
(B) le chlorure de 1,9-diéthylméthyl-thioninium (DEMTC), ou
(F) le sel mixte de chlorure d'éthyl-thioninium et de chlorure de zinc (ETZ) ;
dans lequel le traitement ou la prophylaxie comprend l'administration du composé selon le régime posologique suivant : environ 50 ou environ 75 mg, 3 fois par jour ; environ 50 mg, 4 fois par jour ; ou environ 100 ou environ 125 mg, 2 fois par jour.

13. Composé selon la revendication 12, pour utilisation dans une méthode de traitement ou de prophylaxie de la maladie d'Alzheimer (AD) chez un patient, lequel composé est :
(A) le chlorure d'éthyl-thioninium (ETC),
(B) le chlorure de 1,9-diéthylméthyl-thioninium (DEMTC), ou
(F) le sel mixte de chlorure d'éthyl-thioninium et de chlorure de zinc (ETZ) ;
dans lequel le traitement ou la prophylaxie comprend l'administration du composé selon le régime posologique suivant : environ 50 ou environ 75 mg, 3 fois par jour ; environ 50 mg, 4 fois par jour ; ou environ 100 ou environ 125 mg, 2 fois par jour.

14. Composé selon la revendication 13, lequel composé est :
(A) le chlorure d'éthyl-thioninium (ETC).

15. Composé selon la revendication 12, lequel composé est :
(B) le chlorure de 1,9-diéthylméthyl-thioninium (DEMTC).

16. Composé selon la revendication 12, lequel composé est :
(F) le sel mixte de chlorure d'éthyl-thioninium et de chlorure de zinc (ETZ).

17. Composé selon l'une quelconque des revendications 1 à 16, lequel composé se présente sous la forme d'une unité posologique comprenant le composé en une quantité de 20 à 300 mg et un véhicule, diluant ou excipient pharmaceutiquement acceptable.

18. Composé selon l'une quelconque des revendications 1 à 17, dans lequel le traitement ou la prophylaxie comprend l'administration du composé par voie orale.

19. Composé selon l'une quelconque des revendications 1 à 18, dans lequel le traitement ou la prophylaxie comprend en outre un traitement avec un inhibiteur de cholinestérase, le donépézil, la rivastigmine, la galantamine, un antagoniste de récepteur de NMDA, la mémantine, un agoniste de récepteur muscarinique, ou un inhibiteur de protéine de précurseur d'amyloïde se transformant en β-amyloïde.

20. Utilisation d'un composé dans la fabrication d'un médicament pour utilisation dans une méthode de traitement ou de prophylaxie d'un état de tauopathie chez un patient, ou d'une maladie d'agrégation de la protéine tau chez un patient, dans laquelle le composé est :
(C) le chlorure de 1,9-diméthyléthyl-thioninium (DMETC),
(D) le chlorure de 1,9-diéthyléthyl-thioninium (DEETC),
(I) l'iodure d'éthyl-thioninium (ETI),
(J) le sel mixte d'iodure d'éthyl-thioninium et d'iodure d'hydrogène (ETI.HI), ou
(L) le nitrate d'éthyl-thioninium (ETN).

21. Utilisation d'un composé dans la fabrication d'un médicament pour utilisation dans une méthode de traitement ou de prophylaxie de la maladie d'Alzheimer (AD), de la maladie de Pick, de la paralysie supranucléaire progressive (PSP), de la démence fronto-temporale (FTD), de la démence fronto-temporale avec un parkinsonisme liée au chromosome 17 (FTDP-17), du complexe de désinhibition-démence-parkinsonisme-amyotrophie (DDPAC), de la dégénérescence pallido-ponto-nigrale (PPND), du syndrome de Guam-ALS, de la dégénérescence pallido-nigro-luysienne (PNLD), ou de la dégénérescence cortico-basale (CBD) chez un patient, dans laquelle le composé est :
(C) le chlorure de 1,9-diméthyléthyl-thioninium (DMETC),
(D) le chlorure de 1,9-diéthyléthyl-thioninium (DEETC),
(I) l'iodure d'éthyl-thioninium (ETI),
(J) le sel mixte d'iodure d'éthyl-thioninium et d'iodure d'hydrogène (ETI.HI), ou
(L) le nitrate d'éthyl-thioninium (ETN).

22. Utilisation d'un composé selon la revendication 21 dans la fabrication d'un médicament pour utilisation dans une méthode de traitement ou de prophylaxie de la maladie d'Alzheimer (AD) chez un patient, dans laquelle le composé est :
(C) le chlorure de 1,9-diméthyléthyl-thioninium (DMETC),
(D) le chlorure de 1,9-diéthyléthyl-thioninium (DEETC),
(I) l'iodure d'éthyl-thioninium (ETI),
(J) le sel mixte d'iodure d'éthyl-thioninium et d'iodure d'hydrogène (ETI.HI), ou
(L) le nitrate d'éthyl-thioninium (ETN).

23. Utilisation d'un composé selon la revendication 22, dans laquelle le composé est :
(C) le chlorure de 1,9-diméthyléthyl-thioninium (DMETC).

24. Utilisation d'un composé selon la revendication 22, dans laquelle le composé est :
(D) le chlorure de 1,9-diéthyléthyl-thioninium (DEETC).

25. Utilisation d'un composé selon la revendication 22, dans laquelle le composé est :
(I) l'iodure d'éthyl-thioninium (ETI).

26. Utilisation d'un composé selon la revendication 22, dans laquelle le composé est :
(J) le sel mixte d'iodure d'éthyl-thioninium et d'iodure d'hydrogène (ETI.HI).

27. Utilisation d'un composé selon la revendication 22, dans laquelle le composé est :
(L) le nitrate d'éthyl-thioninium (ETN).

28. Utilisation selon l'une quelconque des revendications 21 à 28, dans laquelle le traitement ou la prophylaxie comprend l'administration du composé selon le régime posologique suivant : environ 50 ou environ 75 mg, 3 ou 4 fois par jour ; ou environ 100 ou environ 125 mg, 2 fois par jour.

29. Utilisation d'un composé dans la fabrication d'un médicament pour utilisation dans une méthode de traitement ou de prophylaxie d'un état de tauopathie chez un patient, ou d'une maladie d'agrégation de la protéine tau chez un patient, dans laquelle le composé est :
(A) le chlorure d'éthyl-thioninium (ETC),
(B) le chlorure de 1,9-diéthylméthyl-thioninium (DEMTC), ou
(F) le sel mixte de chlorure d'éthyl-thioninium et de chlorure de zinc (ETZ) ;
et dans laquelle le traitement ou la prophylaxie comprend l'administration du composé selon le régime posologique suivant : environ 50 ou environ 75 mg, 3 fois par jour ; environ 50 mg, 4 fois par jour ; ou environ 100 ou environ 125 mg, 2 fois par jour.

30. Utilisation d'un composé dans la fabrication d'un médicament pour utilisation dans une méthode de traitement ou de prophylaxie de la maladie d'Alzheimer (AD), de la maladie de Pick, de la paralysie supranucléaire progressive (PSP), de la démence fronto-temporale (FTD), de la démence fronto-temporale avec un parkinsonisme liée au chromosome 17 (FTDP-17), du complexe de désinhibition-démence-parkinsonisme-amyotrophie (DDPAC), de la dégénérescence pallido-ponto-nigrale (PPND), du syndrome de Guam-ALS, de la dégénérescence pallido-nigro-luysienne (PNLD), ou de la dégénérescence cortico-basale (CBD) chez un patient, dans laquelle le composé est :
(A) le chlorure d'éthyl-thioninium (ETC),
(B) le chlorure de 1,9-diéthylméthyl-thioninium (DEMTC), ou
(F) le sel mixte de chlorure d'éthyl-thioninium et de chlorure de zinc (ETZ) ;
et dans laquelle le traitement ou la prophylaxie comprend l'administration du composé selon le régime posologique suivant : environ 50 ou environ 75 mg, 3 fois par jour ; environ 50 mg, 4 fois par jour ; ou environ 100 ou environ 125 mg, 2 fois par jour.

31. Utilisation d'un composé selon la revendication 30 dans la fabrication d'un médicament pour utilisation dans une méthode de traitement ou de prophylaxie de la maladie d'Alzheimer (AD) chez un patient, dans laquelle le composé est :
(A) le chlorure d'éthyl-thioninium (ETC),
(B) le chlorure de 1,9-diéthylméthyl-thioninium (DEMTC), ou
(F) le sel mixte de chlorure d'éthyl-thioninium et de chlorure de zinc (ETZ) ;
et dans laquelle le traitement ou la prophylaxie comprend l'administration du composé selon le régime posologique suivant : environ 50 ou environ 75 mg, 3 fois par jour ; environ 50 mg, 4 fois par jour ; ou environ 100 ou environ 125 mg, 2 fois par jour.

32. Utilisation d'un composé selon la revendication 31, dans laquelle le composé est :
(A) le chlorure d'éthyl-thioninium (ETC).

33. Utilisation d'un composé selon la revendication 31, dans laquelle le composé est :
(B) le chlorure de 1,9-diéthylméthyl-thioninium (DEMTC).

34. Utilisation d'un composé selon la revendication 31, dans laquelle le composé est :
(F) le sel mixte de chlorure d'éthyl-thioninium et de chlorure de zinc (ETZ).

35. Utilisation selon l'une quelconque des revendications 20 à 34, dans laquelle le médicament est une unité posologique comprenant le composé en une quantité de 20 à 300 mg et un véhicule, diluant ou excipient pharmaceutiquement acceptable.

36. Utilisation selon l'une quelconque des revendications 20 à 35, dans laquelle le traitement ou la prophylaxie comprend l'administration du composé par voie orale.

37. Utilisation selon l'une quelconque des revendications 20 à 36, dans laquelle le traitement ou la prophylaxie comprend en outre un traitement avec un inhibiteur de cholinestérase, le donépézil, la rivastigmine, ou la galantamine, un antagoniste de récepteur de NMDA, la mémantine, un agoniste de récepteur muscarinique, ou un inhibiteur de protéine de précurseur d'amyloïde se transformant en β-amyloïde.
